# EUROPEAN PATENT APPLICATION

(11) **EP 4 696 715 A1**
(43) Date of publication of application: **18.02.2026**
(21) Application number: 24788090.9
(22) Date of filing: 10.04.2024
(51) Int. Cl.: C07K 16/46, C12N 15/13, A61K 39/395, A61K 45/00, A61P 35/00, A61P 35/02, A61P 7/06, G01N 33/68, G01N 33/574

(54) **MEDICAL USE OF ANTI-CTLA4 AND ANTI-PD-1 BISPECIFIC ANTIBODY**

(30) Priority: 10.04.2023 CN 202310378536
(71) Applicant: Akeso Pharma Co., Ltd., Guangzhou, Guangdong 510555 (CN)
(72) Inventor: LI, Baiyong, Guangzhou, Guangdong 510799 (CN); XIA, Yu, Guangzhou, Guangdong 510799 (CN); WANG, Zhongmin, Guangzhou, Guangdong 510799 (CN)
(74) Representative: Berggren Oy
(86) International application number: PCT/CN2024/086969
(87) International publication number: WO 2024/212988

(57) **Abstract**

The use of an anti-CTLA4 and anti-PD-1 bispecific antibody in the preparation of a drug for treating or preventing a malignant vulvar tumor. The bispecific antibody comprises a first protein functional region targeting PD-1, and a second protein functional region targeting CTLA4. The first protein functional region is an anti-PD-1 antibody or an antigen-binding fragment thereof, wherein the heavy chain variable region comprises HCDR1-HCDR3 having the amino acid sequences respectively as shown in SEQ ID NOs: 27-29, and the light chain variable region comprises LCDR1-LCDR3 having the amino acid sequences as shown in SEQ ID NOs: 30-32. The second protein functional region is an anti-CTLA4 antibody or an antigen-binding fragment thereof, wherein the heavy chain variable region comprises HCDR1-HCDR3 having the amino acid sequences as shown in SEQ ID NOs: 33-35, and the light chain variable region comprises LCDR1-LCDR3 having the amino acid sequences as shown in SEQ ID NOs: 36-38.

## Description

### CROSS-REFERENCE TO RELATED APPLICATIONS

The present application is based on and claims priority to Chinese Patent Application No. CN202310378536.6 filed on April 10, 2023, which is incorporated herein by reference in its entirety.

### TECHNICAL FIELD

The present disclosure pertains to the field of tumor treatment and molecular immunology and relates to medical use of an anti-CTLA4-anti-PD-1 bispecific antibody. Specifically, the present disclosure relates to use of an anti-CTLA4-anti-PD-1 bispecific antibody in the preparation of a medicament for treating or preventing a malignant tumor of the vulva.

### BACKGROUND

The transmembrane receptor PD-1 (programmed cell death protein-1) is a member of the CD28 gene family, and is expressed in activated T cells, B cells, and myeloid cells. Ligands of PD-1, both PDL1 (programmed cell death 1 ligand 1, or PDL-1) and PDL2 (programmed cell death 1 ligand 2, or PDL-2), are members of the B7 superfamily. PDL1 is expressed in a variety of cells including T cells, B cells, endothelial cells, and epithelial cells, and PDL2 is expressed only in antigen-presenting cells such as dendritic cells and macrophages.

The PD-1/PDL1 signaling pathway plays an important role in regulating immune tolerance, microbial infection, and tumor immune escape. PD-1 is mainly expressed in immune cells such as T cells, and the ligand PDL1 of PD-1 is highly expressed in a plurality of human tumor tissues. Blocking the PD-1/PDL1 signaling pathway may activate inhibited T cells, which thus attack cancer cells. Blocking the PD-1/PDL1 signaling can promote the proliferation of tumor antigen-specific T cells, play a role in killing tumor cells, and further inhibit local tumor growth (Julie R et al., 2012, N Engl J Med., 366:2455-2465). In addition, tumors with high PDL1 expression are associated with cancers that are difficult to detect (Hamanishi et al., 2007, Proc. Natl. Acad. Sci. USA, 104:3360-5). An effective method is the *in-vivo* injection of an anti-PD-1 antibody to modulate the expression of PD-1. Due to the broad-spectrum anti-tumor prospects and surprising efficacy of PD-1 antibodies, it is widely accepted in the industry that antibodies targeting the PD-1 pathway will bring about breakthroughs in the treatment of various tumors, for example, non-small cell lung cancer, renal cell carcinoma, ovarian cancer, melanoma (Homet M. B., Parisi G., et al., 2015, Semin Oncol., 42(3): 466-473), leukemia and anemia (Held SA, Heine A, et al., 2013, Curr Cancer Drug Targets., 13(7): 768-74). Cytotoxic T lymphocyte associated antigen 4 (CTLA4) and CD28 molecules are very similar in aspects of gene structure, chromosome location, sequence homology, and gene expression. Both molecules are receptors of the co-stimulatory molecule B7, and are mainly expressed on the surface of activated T cells. Binding of CTLA4 to B7 can inhibit the activation of mouse and human T cells, and plays a negative regulatory role in T cell activation.

CTLA4 antibodies (or anti-CTLA4 monoclonal antibodies) or CTLA4 ligands can prevent CTLA4 from binding to its natural ligands, thereby blocking the transmission of negative regulatory signals by CTLA4 to T cells and enhancing the reactivity of T cells to various antigens. In this respect, *in-vivo* and *in-vitro* studies are essentially consistent. Currently, there are CTLA4 monoclonal antibodies in clinical trials or approved for treating prostate cancer, bladder cancer, colorectal cancer, gastrointestinal cancer, liver cancer, malignant melanoma, etc. (Grosso JF., Jure-Kunkel MN., 2013, Cancer Immun., 13:5). CTLA4 antibodies can produce a specific therapeutic effect on diseases and show remarkable efficacy, and may be used for supplementing traditional medicines and for exploring new means of gene therapy.

Bispecific antibodies, also known as bifunctional antibodies, are specific drugs that target two different antigens simultaneously, and can be purified and produced by immunomagnetic separation. Alternatively, they can be obtained by genetic engineering. The genetic engineering has corresponding flexibility in aspects of binding site optimization, synthetic form consideration, yield, and the like, thus having certain advantages. Currently, over 45 existence forms have been demonstrated (Dafne Müller, Kontermann R E., 2010, BioDrugs, 24(2): 89-98). A number of developed bispecific antibodies are in the form of IgG-scFv, i.e., the Morrison format (Coloma MJ, Morrison SL., 1997, Nat Biotechnol., 15: 159-163), which has been demonstrated to be one of the ideal forms for the bispecific antibodies because of its similarity to the naturally existing IgG forms and advantages in antibody engineering, expression, and purification (Miller BR, Demarest SJ, et al., 2010, Protein Eng Des Sel, 23: 549-57; Fitzgerald J, Lugovskoy A., 2011, MAbs, 3: 299-309).

Malignant tumor of the vulva is a rare gynecological malignant tumor that accounts for 2%-5% of all malignant tumors of the female reproductive system. Tumors may occur in the skin, mucosa, and accessory tissues of the vulva. Squamous cell carcinoma accounts for the vast majority (more than 80%) of malignant tumors of the vulva, followed by malignant melanoma. Other rare histological types include: basal cell carcinoma, verrucous carcinoma, Paget's disease of the vulva, non-specific adenocarcinoma, Bartholin gland carcinoma, and the like (Rogers Linda J, Cuello Mauricio A, Cancer of the vulva. [J]. Int J Gynaecol Obstet, 2018, null: 4-13). The incidence of malignant tumors of the vulva is on the rise, especially in elderly women aged 75 and older, which may be associated with non-neoplastic epithelial lesions such as lichen sclerosus lesions of the vulva and atypical hyperplasia of epithelial cells caused by advanced age.

Malignant tumors of the vulva can grow along the skin and mucosa to surrounding and deep tissues, involving the urethra, anus, rectum, etc. The vulva is an organ rich in lymphoid tissues. Malignant tumors of the vulva have a relatively high rate of lymphatic metastasis. Therefore, lymph node dissection is often included as part of the surgical procedure. However, this can lead to postoperative complications such as decreased sexual function, lower limb edema, and incontinence of urine and stool. As a result, the quality of life of patients is significantly reduced after surgery (Liu Yudi, Yang Yingjie. Progress of diagnosis and treatment of vulvar cancer. [J]. Contemporary Medicine, 2018, 24(03): 176-179).

Given the toxicity of chemotherapy, it is currently not recommended in clinical practice to use chemotherapy alone as systemic treatment either preoperatively (neoadjuvant) or postoperatively (adjuvant). Chemotherapy is only considered as palliative care for vulvar cancer in cases of metastasis of cancer cells when other treatment methods are not feasible (Merlo S. Modern treatment of vulvar cancer [J]. Radiology and Oncology, 2020, 54(4)). Additionally, many patients fail to achieve long-term disease control after treatment with chemotherapeutic drugs, with the 5-year survival rate remaining very low.

In summary, developing treatment means or the combination administration regimen with lower toxicity and higher efficacy for malignant tumors of the vulva holds significant clinical importance.

### SUMMARY

Through intensive studies and creative efforts, the inventors have found that the anti-CTLA4-anti-PD-1 bispecific antibody involved in the present disclosure has an effective effect in treating or preventing a malignant tumor of the vulva. The present disclosure is detailed below:
One aspect of the present disclosure relates to use of a bispecific antibody or a pharmaceutical composition comprising the bispecific antibody in the preparation of a medicament for treating and/or preventing a malignant tumor of the vulva, wherein the bispecific antibody comprises:
a first protein functional region targeting PD-1 and
a second protein functional region targeting CTLA4,
wherein:
   the first protein functional region is an anti-PD-1 antibody or an antigen-binding fragment thereof, and the anti-PD-1 antibody comprises a heavy chain variable region and a light chain variable region, wherein the heavy chain variable region comprises HCDR1 having the amino acid sequence set forth in SEQ ID NO: 27, HCDR2 having the amino acid sequence set forth in SEQ ID NO: 28, and HCDR3 having the amino acid sequence set forth in SEQ ID NO: 29; and the light chain variable region comprises LCDR1 having the amino acid sequence set forth in SEQ ID NO: 30, LCDR2 having the amino acid sequence set forth in SEQ ID NO: 31, and LCDR3 having the amino acid sequence set forth in SEQ ID NO: 32;
   the second protein functional region is an anti-CTLA4 antibody or an antigen-binding fragment thereof, and the anti-CTLA4 antibody comprises a heavy chain variable region and a light chain variable region, wherein the heavy chain variable region comprises HCDR1 having the amino acid sequence set forth in SEQ ID NO: 33, HCDR2 having the amino acid sequence set forth in SEQ ID NO: 34, and HCDR3 having the amino acid sequence set forth in SEQ ID NO: 35; and the light chain variable region comprises LCDR1 having the amino acid sequence set forth in SEQ ID NO: 36, LCDR2 having the amino acid sequence set forth in SEQ ID NO: 37, and LCDR3 having the amino acid sequence set forth in SEQ ID NO: 38.

In some embodiments of the present disclosure, provided is the use, wherein the heavy chain variable region of the anti-PD-1 antibody has an amino acid sequence selected from SEQ ID NO: 14 and SEQ ID NO: 18, and the light chain variable region of the anti-PD-1 antibody has an amino acid sequence selected from SEQ ID NO: 16 and SEQ ID NO: 20.

In some embodiments of the present disclosure, provided is the use, wherein the heavy chain variable region of the anti-PD-1 antibody has the amino acid sequence set forth in SEQ ID NO: 14, and the light chain variable region of the anti-PD-1 antibody has the amino acid sequence set forth in SEQ ID NO: 16; or
the heavy chain variable region of the anti-PD-1 antibody has the amino acid sequence set forth in SEQ ID NO: 18, and the light chain variable region of the anti-PD-1 antibody has the amino acid sequence set forth in SEQ ID NO: 20. In some embodiments of the present disclosure, provided is the use, wherein
the heavy chain variable region of the anti-CTLA4 antibody has an amino acid sequence selected from SEQ ID NO: 2, SEQ ID NO: 6, SEQ ID NO: 10, SEQ ID NO: 41, and SEQ ID NO: 43, and the light chain variable region of the anti-CTLA4 antibody has an amino acid sequence selected from SEQ ID NO: 4, SEQ ID NO: 8, SEQ ID NO: 12, SEQ ID NO: 42, and SEQ ID NO: 44.

In some embodiments of the present disclosure, provided is the use, wherein
the heavy chain variable region of the anti-CTLA4 antibody has the amino acid sequence set forth in SEQ ID NO: 2, and the light chain variable region of the anti-CTLA4 antibody has the amino acid sequence set forth in SEQ ID NO: 4; the heavy chain variable region of the anti-CTLA4 antibody has the amino acid sequence set forth in SEQ ID NO: 6, and the light chain variable region of the anti-CTLA4 antibody has the amino acid sequence set forth in SEQ ID NO: 8; the heavy chain variable region of the anti-CTLA4 antibody has the amino acid sequence set forth in SEQ ID NO: 10, and the light chain variable region of the anti-CTLA4 antibody has the amino acid sequence set forth in SEQ ID NO: 12; the heavy chain variable region of the anti-CTLA4 antibody has the amino acid sequence set forth in SEQ ID NO: 41, and the light chain variable region of the anti-CTLA4 antibody has the amino acid sequence set forth in SEQ ID NO: 42; or
the heavy chain variable region of the anti-CTLA4 antibody has the amino acid sequence set forth in SEQ ID NO: 43, and the light chain variable region of the anti-CTLA4 antibody has the amino acid sequence set forth in SEQ ID NO: 44. In some embodiments of the present disclosure, provided is the use, wherein the anti-PD-1 antibody or the antigen-binding fragment thereof is selected from Fab, Fab', F(ab')₂, Fd, Fv, dAb, a complementarity determining region fragment, a single chain antibody, a humanized antibody, a chimeric antibody, and a diabody.

In some embodiments of the present disclosure, provided is the use, wherein the anti-CTLA4 antibody or the antigen-binding fragment thereof is selected from Fab, Fab', F(ab')₂, Fd, Fv, dAb, a complementarity determining region fragment, a single chain antibody, a humanized antibody, a chimeric antibody, and a diabody.

In some embodiments of the present disclosure, provided is the use, wherein the first protein functional region is an anti-PD-1 immunoglobulin, and the second protein functional region is an anti-CTLA4 single chain antibody; preferably, the anti-CTLA4 single chain antibody is linked to the N-terminus or the C-terminus of a heavy chain of the anti-PD-1 immunoglobulin.

In some embodiments of the present disclosure, provided is the use, wherein the anti-PD-1 immunoglobulin is of human IgG1 subtype;
preferably, according to the EU numbering system, a heavy chain constant region of the anti-PD-1 immunoglobulin has one of the following mutation combinations:
L234A and L235A;
L234A and G237A;
L235A and G237A; or
L234A, L235A, and G237A.

In some embodiments of the present disclosure, provided is the use, wherein, according to the EU numbering system, the heavy chain constant region of the anti-PD-1 immunoglobulin further has one or more mutations selected from: N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A, and K320A.

In the present disclosure, letters before the position number represent amino acids before mutation, and letters after the position number represent amino acids after mutation, unless otherwise specified.

In some embodiments of the present disclosure, provided is the use, wherein the first protein functional region is an anti-PD-1 single chain antibody, and the second protein functional region is an anti-CTLA4 immunoglobulin; preferably, the anti-PD-1 single chain antibody is linked to the N-terminus or the C-terminus of a heavy chain of the anti-CTLA4 immunoglobulin.

In some embodiments of the present disclosure, provided is the use, wherein
the anti-CTLA4 immunoglobulin is of human IgG1 subtype;
preferably, according to the EU numbering system, a heavy chain constant region of the anti-CTLA4 immunoglobulin has one of the following mutation combinations:
   L234A and L235A;
   L234A and G237A;
   L235A and G237A; or
   L234A, L235A, and G237A.

In some embodiments of the present disclosure, provided is the use, wherein, according to the EU numbering system, the heavy chain constant region of the anti-CTLA4 immunoglobulin further has one or more mutations selected from: N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A, and K320A.

In one or more embodiments of the present disclosure, provided is the use, wherein
a heavy chain variable region of the anti-PD-1 immunoglobulin has an amino acid sequence selected from SEQ ID NO: 14 and SEQ ID NO: 18, and a light chain variable region of the anti-PD-1 immunoglobulin has an amino acid sequence selected from SEQ ID NO: 16 and SEQ ID NO: 20; and, a heavy chain variable region of the anti-CTLA4 single chain antibody has an amino acid sequence selected from SEQ ID NO: 2, SEQ ID NO: 6, SEQ ID NO: 10, SEQ ID NO: 41, and SEQ ID NO: 43, and a light chain variable region of the anti-CTLA4 single chain antibody has an amino acid sequence selected from SEQ ID NO: 4, SEQ ID NO: 8, SEQ ID NO: 12, SEQ ID NO: 42, and SEQ ID NO: 44;
   or
a heavy chain variable region of the anti-CTLA4 immunoglobulin has an amino acid sequence selected from SEQ ID NO: 2, SEQ ID NO: 6, SEQ ID NO: 10, SEQ ID NO: 41, and SEQ ID NO: 43, and a light chain variable region of the anti-CTLA4 immunoglobulin has an amino acid sequence selected from SEQ ID NO: 4, SEQ ID NO: 8, SEQ ID NO: 12, SEQ ID NO: 42, and SEQ ID NO: 44; and, a heavy chain variable region of the anti-PD-1 single chain antibody has an amino acid sequence selected from SEQ ID NO: 14 and SEQ ID NO: 18, and a light chain variable region of the anti-PD-1 single chain antibody has an amino acid sequence selected from SEQ ID NO: 16 and SEQ ID NO: 20.

In one or more embodiments of the present disclosure, provided is the use, wherein the bispecific antibody is selected from any one of the following (1)-(20):
(1) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 2, and a light chain variable region of the anti-CTLA4 single chain antibody has the amino acid sequence set forth in SEQ ID NO: 4;
(2) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 6, and a light chain variable region of the anti-CTLA4 single chain antibody has the amino acid sequence set forth in SEQ ID NO: 8;
(3) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 10, and a light chain variable region of the anti-CTLA4 single chain antibody has the amino acid sequence set forth in SEQ ID NO: 12;
(4) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 2, and a light chain variable region of the anti-CTLA4 single chain antibody has the amino acid sequence set forth in SEQ ID NO: 4;
(5) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 6, and a light chain variable region of the anti-CTLA4 single chain antibody has the amino acid sequence set forth in SEQ ID NO: 8;
(6) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 10, and a light chain variable region of the anti-CTLA4 single chain antibody has the amino acid sequence set forth in SEQ ID NO: 12;
(7) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
(8) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44;
(9) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
(10) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44;
(11) a heavy chain variable region of the anti-CTLA4 immunoglobulin has the amino acid sequence set forth in SEQ ID NO: 2, and a light chain variable region of the anti-CTLA4 immunoglobulin has the amino acid sequence set forth in SEQ ID NO: 4; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(12) a heavy chain variable region of the anti-CTLA4 immunoglobulin has the amino acid sequence set forth in SEQ ID NO: 2, and a light chain variable region of the anti-CTLA4 immunoglobulin has the amino acid sequence set forth in SEQ ID NO: 4; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
(13) a heavy chain variable region of the anti-CTLA4 immunoglobulin has the amino acid sequence set forth in SEQ ID NO: 6, and a light chain variable region of the anti-CTLA4 immunoglobulin has the amino acid sequence set forth in SEQ ID NO: 8; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(14) a heavy chain variable region of the anti-CTLA4 immunoglobulin has the amino acid sequence set forth in SEQ ID NO: 6, and a light chain variable region of the anti-CTLA4 immunoglobulin has the amino acid sequence set forth in SEQ ID NO: 8; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
(15) a heavy chain variable region of the anti-CTLA4 immunoglobulin has the amino acid sequence set forth in SEQ ID NO: 10, and a light chain variable region of the anti-CTLA4 immunoglobulin has the amino acid sequence set forth in SEQ ID NO: 12; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(16) a heavy chain variable region of the anti-CTLA4 immunoglobulin has the amino acid sequence set forth in SEQ ID NO: 10, and a light chain variable region of the anti-CTLA4 immunoglobulin has the amino acid sequence set forth in SEQ ID NO: 12; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
(17) a heavy chain variable region of the anti-CTLA4 immunoglobulin has the amino acid sequence set forth in SEQ ID NO: 41, and a light chain variable region of the anti-CTLA4 immunoglobulin has the amino acid sequence set forth in SEQ ID NO: 42; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(18) a heavy chain variable region of the anti-CTLA4 immunoglobulin has the amino acid sequence set forth in SEQ ID NO: 43, and a light chain variable region of the anti-CTLA4 immunoglobulin has the amino acid sequence set forth in SEQ ID NO: 44; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(19) a heavy chain variable region of the anti-CTLA4 immunoglobulin has the amino acid sequence set forth in SEQ ID NO: 41, and a light chain variable region of the anti-CTLA4 immunoglobulin has the amino acid sequence set forth in SEQ ID NO: 42; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
   and
(20) a heavy chain variable region of the anti-CTLA4 immunoglobulin has the amino acid sequence set forth in SEQ ID NO: 43, and a light chain variable region of the anti-CTLA4 immunoglobulin has the amino acid sequence set forth in SEQ ID NO: 44; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20.

In some embodiments of the present disclosure, provided is the use, wherein a heavy chain of the anti-PD-1 antibody has the amino acid sequence set forth in SEQ ID NO: 40, and a light chain of the anti-PD-1 antibody has the amino acid sequence set forth in SEQ ID NO: 24; or
a heavy chain of the anti-PD-1 antibody has the amino acid sequence set forth in SEQ ID NO: 22, and a light chain of the anti-PD-1 antibody has the amino acid sequence set forth in SEQ ID NO: 24.

In some embodiments of the present disclosure, provided is the use, wherein the first protein functional region is linked to the second protein functional region either directly or via a linker (also known as linker fragment); and/or the heavy chain variable region of the single chain antibody is linked to the light chain variable region of the single chain antibody either directly or via a linker. In some embodiments of the present disclosure, provided is the use, wherein the linker is independently the polypeptide set forth in SEQ ID NO: 47 (GGGGS) or a polypeptide formed by concatenating a plurality of (e.g., 2, 3, 4, 5, or 6) polypeptides set forth in SEQ ID NO: 47;
preferably, the linker is independently a polypeptide having one or more glycines linked to the C-terminus of the polypeptide set forth in SEQ ID NO: 47 or a polypeptide formed by concatenating a plurality of (e.g., 2, 3, 4, 5, or 6) polypeptides set forth in SEQ ID NO: 47.

In some embodiments of the present disclosure, provided is the use, wherein the first protein functional region and the second protein functional region are each independently 1, 2, or more in number.

In some embodiments of the present disclosure, provided is the use, wherein the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody;
a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26; preferably, the first linker and the second linker both have the amino acid sequence set forth in SEQ ID NO: 26.

In some embodiments of the present disclosure, provided is the use, wherein the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody; a heavy chain of the anti-PD-1 immunoglobulin has the amino acid sequence set forth in SEQ ID NO: 22, and a light chain of the anti-PD-1 immunoglobulin has the amino acid sequence set forth in SEQ ID NO: 24;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26; preferably, the first linker and the second linker both have the amino acid sequence set forth in SEQ ID NO: 26.

The present disclosure also relates to use of a bispecific antibody or a pharmaceutical composition comprising the bispecific antibody in the preparation of a medicament for treating or preventing a malignant tumor of the vulva, wherein the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody; a heavy chain of the anti-PD-1 immunoglobulin has the amino acid sequence set forth in SEQ ID NO: 40, and a light chain of the anti-PD-1 immunoglobulin has the amino acid sequence set forth in SEQ ID NO: 24;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26; preferably, the first linker and the second linker both have the amino acid sequence set forth in SEQ ID NO: 26.

In one or more embodiments of the present disclosure, provided is the use, wherein the bispecific antibody is in the form of IgG-scFv, i.e., the Morrison format.

In one or more embodiments of the present disclosure, provided is the use, wherein the single chain antibody is linked to the C-terminus of the heavy chain of the immunoglobulin. Since an immunoglobulin has two heavy chains, two single chain antibody molecules are linked to one immunoglobulin molecule. Preferably, the two single chain antibody molecules are identical. Preferably, the single chain antibody is linked to the C-terminus of the heavy chain of the immunoglobulin by forming an amide bond via a linker.

In one or more embodiments of the present disclosure, the constant regions of the immunoglobulin (e.g., anti-PD-1 immunoglobulin or anti-CTLA4 immunoglobulin) are humanized. For example, the heavy chain constant region is Ig gamma-1 chain C region, ACCESSION: P01857, and the light chain constant region is Ig kappa chain C region, ACCESSION: P01834.

In one or more embodiments of the present disclosure, provided is the use, wherein the bispecific antibody is a humanized antibody.

In one or more embodiments of the present disclosure, provided is the use, wherein the bispecific antibody is an anti-PD-1-anti-CTLA4 bispecific antibody.

In some embodiments of the present disclosure, provided is the use, wherein the malignant tumor of the vulva is one or more selected from squamous cell carcinoma of the vulva, malignant melanoma of the vulva, basal cell carcinoma of the vulva, verrucous carcinoma of the vulva, Paget's disease of the vulva, sarcoma of the vulva, adenocarcinoma of the vulva, and Bartholin gland carcinoma of the vulva;
preferably, the malignant tumor of the vulva is a recurrent malignant tumor or a metastatic malignant tumor of the vulva;
preferably, the malignant tumor of the vulva is one or more of MSI-H/dMMR malignant tumors of the vulva.

In some embodiments of the present disclosure, provided is the use, wherein the pharmaceutical composition comprises an effective amount of the bispecific antibody according to any one of the embodiments of the present disclosure.

In some embodiments of the present disclosure, provided is the use, wherein the pharmaceutical composition further comprises one or more pharmaceutically acceptable auxiliary materials.

In some embodiments of the present disclosure, provided is the use, wherein the bispecific antibody according to any one of the embodiments of the present disclosure is the only active ingredient in the pharmaceutical composition.

In some embodiments of the present disclosure, provided is the use, wherein the pharmaceutical composition consists of the bispecific antibody according to any one of the embodiments of the present disclosure and one or more pharmaceutically acceptable auxiliary materials.

In some embodiments of the present disclosure, provided is the use, wherein, based on the mass of the bispecific antibody, the pharmaceutical composition has a unit dose of 100 mg-1000 mg, 200 mg-800 mg, 200 mg-500 mg, 300 mg-600 mg, 400 mg-500 mg, or 450 mg.

In some embodiments of the present disclosure, provided is the use, wherein the pharmaceutical composition further comprises one or more anti-tumor chemotherapeutic drugs in effective amounts.

In some embodiments of the present disclosure, provided is the use, wherein active ingredients of the pharmaceutical composition consist of an effective amount of the bispecific antibody according to any one of the embodiments of the present disclosure and one or more anti-tumor chemotherapeutic drugs in effective amounts.

In some embodiments of the present disclosure, provided is the use, wherein the pharmaceutical composition consists of an effective amount of the bispecific antibody according to any one of the embodiments of the present disclosure, one or more anti-tumor chemotherapeutic drugs in effective amounts, and one or more pharmaceutically acceptable auxiliary materials.

In some embodiments of the present disclosure, provided is the use, wherein the pharmaceutical composition is an injection; preferably, the pharmaceutical composition is administered by intravenous drip infusion, intravenous injection, subcutaneous injection, or intraperitoneal injection.

Provided is the bispecific antibody or the pharmaceutical composition comprising the bispecific antibody according to any one of the embodiments of the present disclosure for use in treating and/or preventing a malignant tumor of the vulva, wherein
preferably, the malignant tumor of the vulva is one or more selected from squamous cell carcinoma of the vulva, malignant melanoma of the vulva, basal cell carcinoma of the vulva, verrucous carcinoma of the vulva, Paget's disease of the vulva, sarcoma of the vulva, adenocarcinoma of the vulva, and Bartholin gland carcinoma of the vulva;
preferably, the malignant tumor of the vulva is a recurrent malignant tumor or a metastatic malignant tumor of the vulva;
preferably, the malignant tumor of the vulva is an MSI-H/dMMR malignant tumor of the vulva.

The present disclosure also relates to a bispecific antibody or a pharmaceutical composition comprising the bispecific antibody for use in treating or preventing a malignant tumor of the vulva, wherein the bispecific antibody comprises:
a first protein functional region targeting PD-1 and
a second protein functional region targeting CTLA4,
wherein:
   the first protein functional region is an anti-PD-1 antibody or an antigen-binding fragment thereof, and the anti-PD-1 antibody comprises a heavy chain variable region and a light chain variable region, wherein the heavy chain variable region comprises HCDR1 having the amino acid sequence set forth in SEQ ID NO: 27, HCDR2 having the amino acid sequence set forth in SEQ ID NO: 28, and HCDR3 having the amino acid sequence set forth in SEQ ID NO: 29; and the light chain variable region comprises LCDR1 having the amino acid sequence set forth in SEQ ID NO: 30, LCDR2 having the amino acid sequence set forth in SEQ ID NO: 31, and LCDR3 having the amino acid sequence set forth in SEQ ID NO: 32;
   the second protein functional region is an anti-CTLA4 antibody or an antigen-binding fragment thereof, and the anti-CTLA4 antibody comprises a heavy chain variable region and a light chain variable region, wherein the heavy chain variable region comprises HCDR1 having the amino acid sequence set forth in SEQ ID NO: 33, HCDR2 having the amino acid sequence set forth in SEQ ID NO: 34, and HCDR3 having the amino acid sequence set forth in SEQ ID NO: 35; and the light chain variable region comprises LCDR1 having the amino acid sequence set forth in SEQ ID NO: 36, LCDR2 having the amino acid sequence set forth in SEQ ID NO: 37, and LCDR3 having the amino acid sequence set forth in SEQ ID NO: 38.

In some embodiments of the present disclosure, provided is the bispecific antibody or the pharmaceutical composition comprising the bispecific antibody, wherein
the heavy chain variable region of the anti-PD-1 antibody has an amino acid sequence selected from SEQ ID NO: 14 and SEQ ID NO: 18, and the light chain variable region of the anti-PD-1 antibody has an amino acid sequence selected from SEQ ID NO: 16 and SEQ ID NO: 20;
preferably, the heavy chain variable region of the anti-PD-1 antibody has the amino acid sequence set forth in SEQ ID NO: 14, and the light chain variable region of the anti-PD-1 antibody has the amino acid sequence set forth in SEQ ID NO: 16; or
preferably, the heavy chain variable region of the anti-PD-1 antibody has the amino acid sequence set forth in SEQ ID NO: 18, and the light chain variable region of the anti-PD-1 antibody has the amino acid sequence set forth in SEQ ID NO: 20.

In some embodiments of the present disclosure, provided is the bispecific antibody or the pharmaceutical composition comprising the bispecific antibody,
wherein
the heavy chain variable region of the anti-CTLA4 antibody has an amino acid sequence selected from SEQ ID NO: 2, SEQ ID NO: 6, SEQ ID NO: 10, SEQ ID NO: 41, and SEQ ID NO: 43, and the light chain variable region of the anti-CTLA4 antibody has an amino acid sequence selected from SEQ ID NO: 4, SEQ ID NO: 8, SEQ ID NO: 12, SEQ ID NO: 42, and SEQ ID NO: 44;
preferably:
   the heavy chain variable region of the anti-CTLA4 antibody has the amino acid sequence set forth in SEQ ID NO: 2, and the light chain variable region of the anti-CTLA4 antibody has the amino acid sequence set forth in SEQ ID NO: 4;
   the heavy chain variable region of the anti-CTLA4 antibody has the amino acid sequence set forth in SEQ ID NO: 6, and the light chain variable region of the anti-CTLA4 antibody has the amino acid sequence set forth in SEQ ID NO: 8; the heavy chain variable region of the anti-CTLA4 antibody has the amino acid sequence set forth in SEQ ID NO: 10, and the light chain variable region of the anti-CTLA4 antibody has the amino acid sequence set forth in SEQ ID NO: 12; the heavy chain variable region of the anti-CTLA4 antibody has the amino acid sequence set forth in SEQ ID NO: 41, and the light chain variable region of the anti-CTLA4 antibody has the amino acid sequence set forth in SEQ ID NO: 42; or
   the heavy chain variable region of the anti-CTLA4 antibody has the amino acid sequence set forth in SEQ ID NO: 43, and the light chain variable region of the anti-CTLA4 antibody has the amino acid sequence set forth in SEQ ID NO: 44. In some embodiments of the present disclosure, provided is the bispecific antibody or the pharmaceutical composition comprising the bispecific antibody, wherein
   the first protein functional region is an anti-PD-1 immunoglobulin, and the second protein functional region is an anti-CTLA4 single chain antibody; preferably, the anti-CTLA4 single chain antibody is linked to the N-terminus or the C-terminus of a heavy chain of the anti-PD-1 immunoglobulin; preferably, the anti-PD-1 immunoglobulin is of human IgG1 subtype; preferably, according to the EU numbering system, a heavy chain constant region of the anti-PD-1 immunoglobulin has one of the following mutation combinations:
      L234A and L235A;
      L234A and G237A;
      L235A and G237A; or
      L234A, L235A, and G237A.

In some embodiments of the present disclosure, provided is the bispecific antibody or the pharmaceutical composition comprising the bispecific antibody, wherein the first protein functional region is an anti-PD-1 single chain antibody, and the second protein functional region is an anti-CTLA4 immunoglobulin; preferably, the anti-PD-1 single chain antibody is linked to the N-terminus or the C-terminus of a heavy chain of the anti-CTLA4 immunoglobulin; preferably, the anti-CTLA4 immunoglobulin is of human IgG1 subtype; preferably, according to the EU numbering system, a heavy chain constant region of the immunoglobulin has one of the following mutation combinations:
L234A and L235A;
L234A and G237A;
L235A and G237A; or
L234A, L235A, and G237A.

In some embodiments of the present disclosure, provided is the bispecific antibody or the pharmaceutical composition comprising the bispecific antibody, wherein
a heavy chain variable region of the anti-PD-1 immunoglobulin has an amino acid sequence selected from SEQ ID NO: 14 and SEQ ID NO: 18, and a light chain variable region of the anti-PD-1 immunoglobulin has an amino acid sequence selected from SEQ ID NO: 16 and SEQ ID NO: 20; and, a heavy chain variable region of the anti-CTLA4 single chain antibody has an amino acid sequence selected from SEQ ID NO: 2, SEQ ID NO: 6, SEQ ID NO: 10, SEQ ID NO: 41, and SEQ ID NO: 43, and a light chain variable region of the anti-CTLA4 single chain antibody has an amino acid sequence selected from SEQ ID NO: 4, SEQ ID NO: 8, SEQ ID NO: 12, SEQ ID NO: 42, and SEQ ID NO: 44;
   or
a heavy chain variable region of the anti-CTLA4 immunoglobulin has an amino acid sequence selected from SEQ ID NO: 2, SEQ ID NO: 6, SEQ ID NO: 10, SEQ ID NO: 41, and SEQ ID NO: 43, and a light chain variable region of the anti-CTLA4 immunoglobulin has an amino acid sequence selected from SEQ ID NO: 4, SEQ ID NO: 8, SEQ ID NO: 12, SEQ ID NO: 42, and SEQ ID NO: 44; and, a heavy chain variable region of the anti-PD-1 single chain antibody has an amino acid sequence selected from SEQ ID NO: 14 and SEQ ID NO: 18, and a light chain variable region of the anti-PD-1 single chain antibody has an amino acid sequence selected from SEQ ID NO: 16 and SEQ ID NO: 20; preferably, the bispecific antibody is selected from any one of the following (1)-(20):
   (1) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 2, and a light chain variable region of the anti-CTLA4 single chain antibody has the amino acid sequence set forth in SEQ ID NO: 4;
   (2) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 6, and a light chain variable region of the anti-CTLA4 single chain antibody has the amino acid sequence set forth in SEQ ID NO: 8;
   (3) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 10, and a light chain variable region of the anti-CTLA4 single chain antibody has the amino acid sequence set forth in SEQ ID NO: 12;
   (4) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 2, and a light chain variable region of the anti-CTLA4 single chain antibody has the amino acid sequence set forth in SEQ ID NO: 4;
   (5) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 6, and a light chain variable region of the anti-CTLA4 single chain antibody has the amino acid sequence set forth in SEQ ID NO: 8;
   (6) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 10, and a light chain variable region of the anti-CTLA4 single chain antibody has the amino acid sequence set forth in SEQ ID NO: 12;
   (7) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
   (8) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44;
   (9) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
   (10) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44;
   (11) a heavy chain variable region of the anti-CTLA4 immunoglobulin has the amino acid sequence set forth in SEQ ID NO: 2, and a light chain variable region of the anti-CTLA4 immunoglobulin has the amino acid sequence set forth in SEQ ID NO: 4; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
   (12) a heavy chain variable region of the anti-CTLA4 immunoglobulin has the amino acid sequence set forth in SEQ ID NO: 2, and a light chain variable region of the anti-CTLA4 immunoglobulin has the amino acid sequence set forth in SEQ ID NO: 4; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
   (13) a heavy chain variable region of the anti-CTLA4 immunoglobulin has the amino acid sequence set forth in SEQ ID NO: 6, and a light chain variable region of the anti-CTLA4 immunoglobulin has the amino acid sequence set forth in SEQ ID NO: 8; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
   (14) a heavy chain variable region of the anti-CTLA4 immunoglobulin has the amino acid sequence set forth in SEQ ID NO: 6, and a light chain variable region of the anti-CTLA4 immunoglobulin has the amino acid sequence set forth in SEQ ID NO: 8; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
   (15) a heavy chain variable region of the anti-CTLA4 immunoglobulin has the amino acid sequence set forth in SEQ ID NO: 10, and a light chain variable region of the anti-CTLA4 immunoglobulin has the amino acid sequence set forth in SEQ ID NO: 12; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
   (16) a heavy chain variable region of the anti-CTLA4 immunoglobulin has the amino acid sequence set forth in SEQ ID NO: 10, and a light chain variable region of the anti-CTLA4 immunoglobulin has the amino acid sequence set forth in SEQ ID NO: 12; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
   (17) a heavy chain variable region of the anti-CTLA4 immunoglobulin has the amino acid sequence set forth in SEQ ID NO: 41, and a light chain variable region of the anti-CTLA4 immunoglobulin has the amino acid sequence set forth in SEQ ID NO: 42; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
   (18) a heavy chain variable region of the anti-CTLA4 immunoglobulin has the amino acid sequence set forth in SEQ ID NO: 43, and a light chain variable region of the anti-CTLA4 immunoglobulin has the amino acid sequence set forth in SEQ ID NO: 44; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
   (19) a heavy chain variable region of the anti-CTLA4 immunoglobulin has the amino acid sequence set forth in SEQ ID NO: 41, and a light chain variable region of the anti-CTLA4 immunoglobulin has the amino acid sequence set forth in SEQ ID NO: 42; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
      and
   (20) a heavy chain variable region of the anti-CTLA4 immunoglobulin has the amino acid sequence set forth in SEQ ID NO: 43, and a light chain variable region of the anti-CTLA4 immunoglobulin has the amino acid sequence set forth in SEQ ID NO: 44; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20.

In some embodiments of the present disclosure, provided is the bispecific antibody or the pharmaceutical composition comprising the bispecific antibody, wherein
a heavy chain of the anti-PD-1 antibody has the amino acid sequence set forth in SEQ ID NO: 40, and a light chain of the anti-PD-1 antibody has the amino acid sequence set forth in SEQ ID NO: 24; or
a heavy chain of the anti-PD-1 antibody has the amino acid sequence set forth in SEQ ID NO: 22, and a light chain of the anti-PD-1 antibody has the amino acid sequence set forth in SEQ ID NO: 24.

In some embodiments of the present disclosure, provided is the bispecific antibody or the pharmaceutical composition comprising the bispecific antibody, wherein the first protein functional region is linked to the second protein functional region either directly or via a linker; and/or the heavy chain variable region of the single chain antibody is linked to the light chain variable region of the single chain antibody either directly or via a linker;
preferably, the linker is independently the polypeptide set forth in SEQ ID NO: 47 or a polypeptide formed by concatenating a plurality of (e.g., 2, 3, 4, 5, or 6) polypeptides set forth in SEQ ID NO: 47;
preferably, the linker is independently a polypeptide having one or more glycines linked to the C-terminus of the polypeptide set forth in SEQ ID NO: 47 or a polypeptide formed by concatenating a plurality of (e.g., 2, 3, 4, 5, or 6) polypeptides set forth in SEQ ID NO: 47;
preferably, the first protein functional region and the second protein functional region are independently 1, 2, or more in number.

In some embodiments of the present disclosure, provided is the bispecific antibody or the pharmaceutical composition comprising the bispecific antibody, wherein
the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody; a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26; preferably, the first linker and the second linker both have the amino acid sequence set forth in SEQ ID NO: 26.

In some embodiments of the present disclosure, provided is the bispecific antibody or the pharmaceutical composition comprising the bispecific antibody, wherein
the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody; a heavy chain of the anti-PD-1 immunoglobulin has the amino acid sequence set forth in SEQ ID NO: 22, and a light chain of the anti-PD-1 immunoglobulin has the amino acid sequence set forth in SEQ ID NO: 24;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26; preferably, the first linker and the second linker both have the amino acid sequence set forth in SEQ ID NO: 26.

The present disclosure also relates to a bispecific antibody or a pharmaceutical composition comprising the bispecific antibody for use in treating or preventing a malignant tumor of the vulva, wherein the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody;
a heavy chain of the anti-PD-1 immunoglobulin has the amino acid sequence set forth in SEQ ID NO: 40, and a light chain of the anti-PD-1 immunoglobulin has the amino acid sequence set forth in SEQ ID NO: 24;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26; preferably, the first linker and the second linker both have the amino acid sequence set forth in SEQ ID NO: 26.

In some embodiments of the present disclosure, provided is the bispecific antibody or the pharmaceutical composition comprising the bispecific antibody, wherein the malignant tumor of the vulva is one or more selected from squamous cell carcinoma of the vulva, malignant melanoma of the vulva, basal cell carcinoma of the vulva, verrucous carcinoma of the vulva, Paget's disease of the vulva, sarcoma of the vulva, adenocarcinoma of the vulva, and Bartholin gland carcinoma of the vulva;
preferably, the malignant tumor of the vulva is a recurrent malignant tumor or a metastatic malignant tumor of the vulva;
preferably, the malignant tumor of the vulva is an MSI-H/dMMR malignant tumor of the vulva.

In some embodiments of the present disclosure, provided is the bispecific antibody or the pharmaceutical composition comprising the bispecific antibody, wherein, based on the mass of the bispecific antibody, the pharmaceutical composition has a unit dose of 100 mg-1000 mg, 200 mg-800 mg, 200 mg-500 mg, 300 mg-600 mg, 400 mg-500 mg, or 450 mg.

In some embodiments of the present disclosure, provided is the bispecific antibody or the pharmaceutical composition comprising the bispecific antibody, wherein the pharmaceutical composition further comprises one or more pharmaceutically acceptable auxiliary materials;
preferably, the pharmaceutical composition further comprises one or more anti-tumor chemotherapeutic drugs in effective amounts.

In some embodiments of the present disclosure, provided is the bispecific antibody or the pharmaceutical composition comprising the bispecific antibody, wherein the pharmaceutical composition is an injection; preferably, the pharmaceutical composition is administered by intravenous drip infusion, intravenous injection, subcutaneous injection, or intraperitoneal injection.

Yet another aspect of the present disclosure relates to a method for treating or preventing a malignant tumor of the vulva, which comprises a step of administering to a subject a therapeutically effective amount of a bispecific antibody, wherein the bispecific antibody is the bispecific antibody according to any one of the above embodiments;
the malignant tumor of the vulva is one or more selected from squamous cell carcinoma of the vulva, malignant melanoma of the vulva, basal cell carcinoma of the vulva, verrucous carcinoma of the vulva, Paget's disease of the vulva, sarcoma of the vulva, adenocarcinoma of the vulva, and Bartholin gland carcinoma of the vulva;
preferably, the malignant tumor of the vulva is a recurrent malignant tumor or a metastatic malignant tumor of the vulva;
preferably, the malignant tumor of the vulva is one or more of MSI-H/dMMR malignant tumors of the vulva;
preferably, the method further comprises a step of co-administering one or more chemotherapeutic drugs for treating a tumor.

The present disclosure also relates to a method for treating or preventing a malignant tumor of the vulva, which comprises a step of administering to a subject in need an effective amount of a bispecific antibody or a pharmaceutical composition comprising the bispecific antibody, wherein the bispecific antibody comprises:
a first protein functional region targeting PD-1 and
a second protein functional region targeting CTLA4,
wherein:
   the first protein functional region is an anti-PD-1 antibody or an antigen-binding fragment thereof, and the anti-PD-1 antibody comprises a heavy chain variable region and a light chain variable region, wherein the heavy chain variable region comprises HCDR1 having the amino acid sequence set forth in SEQ ID NO: 27, HCDR2 having the amino acid sequence set forth in SEQ ID NO: 28, and HCDR3 having the amino acid sequence set forth in SEQ ID NO: 29; and the light chain variable region comprises LCDR1 having the amino acid sequence set forth in SEQ ID NO: 30, LCDR2 having the amino acid sequence set forth in SEQ ID NO: 31, and LCDR3 having the amino acid sequence set forth in SEQ ID NO: 32;
   the second protein functional region is an anti-CTLA4 antibody or an antigen-binding fragment thereof, and the anti-CTLA4 antibody comprises a heavy chain variable region and a light chain variable region, wherein the heavy chain variable region comprises HCDR1 having the amino acid sequence set forth in SEQ ID NO: 33, HCDR2 having the amino acid sequence set forth in SEQ ID NO: 34, and HCDR3 having the amino acid sequence set forth in SEQ ID NO: 35; and the light chain variable region comprises LCDR1 having the amino acid sequence set forth in SEQ ID NO: 36, LCDR2 having the amino acid sequence set forth in SEQ ID NO: 37, and LCDR3 having the amino acid sequence set forth in SEQ ID NO: 38.

In some embodiments of the present disclosure, provided is the method for treating or preventing a malignant tumor of the vulva, wherein
the heavy chain variable region of the anti-PD-1 antibody has an amino acid sequence selected from SEQ ID NO: 14 and SEQ ID NO: 18, and the light chain variable region of the anti-PD-1 antibody has an amino acid sequence selected from SEQ ID NO: 16 and SEQ ID NO: 20;
preferably, the heavy chain variable region of the anti-PD-1 antibody has the amino acid sequence set forth in SEQ ID NO: 14, and the light chain variable region of the anti-PD-1 antibody has the amino acid sequence set forth in SEQ ID NO: 16; or
preferably, the heavy chain variable region of the anti-PD-1 antibody has the amino acid sequence set forth in SEQ ID NO: 18, and the light chain variable region of the anti-PD-1 antibody has the amino acid sequence set forth in SEQ ID NO: 20.

In some embodiments of the present disclosure, provided is the method for treating or preventing a malignant tumor of the vulva, wherein
the heavy chain variable region of the anti-CTLA4 antibody has an amino acid sequence selected from SEQ ID NO: 2, SEQ ID NO: 6, SEQ ID NO: 10, SEQ ID NO: 41, and SEQ ID NO: 43, and the light chain variable region of the anti-CTLA4 antibody has an amino acid sequence selected from SEQ ID NO: 4, SEQ ID NO: 8, SEQ ID NO: 12, SEQ ID NO: 42, and SEQ ID NO: 44;
preferably:
   the heavy chain variable region of the anti-CTLA4 antibody has the amino acid sequence set forth in SEQ ID NO: 2, and the light chain variable region of the anti-CTLA4 antibody has the amino acid sequence set forth in SEQ ID NO: 4;
   the heavy chain variable region of the anti-CTLA4 antibody has the amino acid sequence set forth in SEQ ID NO: 6, and the light chain variable region of the anti-CTLA4 antibody has the amino acid sequence set forth in SEQ ID NO: 8;
   the heavy chain variable region of the anti-CTLA4 antibody has the amino acid sequence set forth in SEQ ID NO: 10, and the light chain variable region of the anti-CTLA4 antibody has the amino acid sequence set forth in SEQ ID NO: 12;
   the heavy chain variable region of the anti-CTLA4 antibody has the amino acid sequence set forth in SEQ ID NO: 41, and the light chain variable region of the anti-CTLA4 antibody has the amino acid sequence set forth in SEQ ID NO: 42;
      or
   the heavy chain variable region of the anti-CTLA4 antibody has the amino acid sequence set forth in SEQ ID NO: 43, and the light chain variable region of the anti-CTLA4 antibody has the amino acid sequence set forth in SEQ ID NO: 44.

In some embodiments of the present disclosure, provided is the method for treating or preventing a malignant tumor of the vulva, wherein
the first protein functional region is an anti-PD-1 immunoglobulin, and the second protein functional region is an anti-CTLA4 single chain antibody;
preferably, the anti-CTLA4 single chain antibody is linked to the N-terminus or the C-terminus of a heavy chain of the anti-PD-1 immunoglobulin;
preferably, the anti-PD-1 immunoglobulin is of human IgG1 subtype;
preferably, according to the EU numbering system, a heavy chain constant region of the anti-PD-1 immunoglobulin has one of the following mutation combinations:
   L234A and L235A;
   L234A and G237A;
   L235A and G237A; or
   L234A, L235A, and G237A.

In some embodiments of the present disclosure, provided is the method for treating or preventing a malignant tumor of the vulva, wherein
the first protein functional region is an anti-PD-1 single chain antibody, and the second protein functional region is an anti-CTLA4 immunoglobulin;
preferably, the anti-PD-1 single chain antibody is linked to the N-terminus or the C-terminus of a heavy chain of the anti-CTLA4 immunoglobulin;
preferably, the anti-CTLA4 immunoglobulin is of human IgG1 subtype;
preferably, according to the EU numbering system, a heavy chain constant region of the immunoglobulin has one of the following mutation combinations:
   L234A and L235A;
   L234A and G237A;
   L235A and G237A; or
   L234A, L235A, and G237A.

In some embodiments of the present disclosure, provided is the method for treating or preventing a malignant tumor of the vulva, wherein
a heavy chain variable region of the anti-PD-1 immunoglobulin has an amino acid sequence selected from SEQ ID NO: 14 and SEQ ID NO: 18, and a light chain variable region of the anti-PD-1 immunoglobulin has an amino acid sequence selected from SEQ ID NO: 16 and SEQ ID NO: 20; and, a heavy chain variable region of the anti-CTLA4 single chain antibody has an amino acid sequence selected from SEQ ID NO: 2, SEQ ID NO: 6, SEQ ID NO: 10, SEQ ID NO: 41, and SEQ ID NO: 43, and a light chain variable region of the anti-CTLA4 single chain antibody has an amino acid sequence selected from SEQ ID NO: 4, SEQ ID NO: 8, SEQ ID NO: 12, SEQ ID NO: 42, and SEQ ID NO: 44;
   or
a heavy chain variable region of the anti-CTLA4 immunoglobulin has an amino acid sequence selected from SEQ ID NO: 2, SEQ ID NO: 6, SEQ ID NO: 10, SEQ ID NO: 41, and SEQ ID NO: 43, and a light chain variable region of the anti-CTLA4 immunoglobulin has an amino acid sequence selected from SEQ ID NO: 4, SEQ ID NO: 8, SEQ ID NO: 12, SEQ ID NO: 42, and SEQ ID NO: 44; and, a heavy chain variable region of the anti-PD-1 single chain antibody has an amino acid sequence selected from SEQ ID NO: 14 and SEQ ID NO: 18, and a light chain variable region of the anti-PD-1 single chain antibody has an amino acid sequence selected from SEQ ID NO: 16 and SEQ ID NO: 20;
preferably, the bispecific antibody is selected from any one of the following (1)-(20):
   (1) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 2, and a light chain variable region of the anti-CTLA4 single chain antibody has the amino acid sequence set forth in SEQ ID NO: 4;
   (2) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 6, and a light chain variable region of the anti-CTLA4 single chain antibody has the amino acid sequence set forth in SEQ ID NO: 8;
   (3) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 10, and a light chain variable region of the anti-CTLA4 single chain antibody has the amino acid sequence set forth in SEQ ID NO: 12;
   (4) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 2, and a light chain variable region of the anti-CTLA4 single chain antibody has the amino acid sequence set forth in SEQ ID NO: 4;
   (5) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 6, and a light chain variable region of the anti-CTLA4 single chain antibody has the amino acid sequence set forth in SEQ ID NO: 8;
   (6) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 10, and a light chain variable region of the anti-CTLA4 single chain antibody has the amino acid sequence set forth in SEQ ID NO: 12;
   (7) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
   (8) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44;
   (9) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
   (10) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44;
   (11) a heavy chain variable region of the anti-CTLA4 immunoglobulin has the amino acid sequence set forth in SEQ ID NO: 2, and a light chain variable region of the anti-CTLA4 immunoglobulin has the amino acid sequence set forth in SEQ ID NO: 4; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
   (12) a heavy chain variable region of the anti-CTLA4 immunoglobulin has the amino acid sequence set forth in SEQ ID NO: 2, and a light chain variable region of the anti-CTLA4 immunoglobulin has the amino acid sequence set forth in SEQ ID NO: 4; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
   (13) a heavy chain variable region of the anti-CTLA4 immunoglobulin has the amino acid sequence set forth in SEQ ID NO: 6, and a light chain variable region of the anti-CTLA4 immunoglobulin has the amino acid sequence set forth in SEQ ID NO: 8; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
   (14) a heavy chain variable region of the anti-CTLA4 immunoglobulin has the amino acid sequence set forth in SEQ ID NO: 6, and a light chain variable region of the anti-CTLA4 immunoglobulin has the amino acid sequence set forth in SEQ ID NO: 8; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
   (15) a heavy chain variable region of the anti-CTLA4 immunoglobulin has the amino acid sequence set forth in SEQ ID NO: 10, and a light chain variable region of the anti-CTLA4 immunoglobulin has the amino acid sequence set forth in SEQ ID NO: 12; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
   (16) a heavy chain variable region of the anti-CTLA4 immunoglobulin has the amino acid sequence set forth in SEQ ID NO: 10, and a light chain variable region of the anti-CTLA4 immunoglobulin has the amino acid sequence set forth in SEQ ID NO: 12; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
   (17) a heavy chain variable region of the anti-CTLA4 immunoglobulin has the amino acid sequence set forth in SEQ ID NO: 41, and a light chain variable region of the anti-CTLA4 immunoglobulin has the amino acid sequence set forth in SEQ ID NO: 42; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
   (18) a heavy chain variable region of the anti-CTLA4 immunoglobulin has the amino acid sequence set forth in SEQ ID NO: 43, and a light chain variable region of the anti-CTLA4 immunoglobulin has the amino acid sequence set forth in SEQ ID NO: 44; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
   (19) a heavy chain variable region of the anti-CTLA4 immunoglobulin has the amino acid sequence set forth in SEQ ID NO: 41, and a light chain variable region of the anti-CTLA4 immunoglobulin has the amino acid sequence set forth in SEQ ID NO: 42; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
      and
   (20) a heavy chain variable region of the anti-CTLA4 immunoglobulin has the amino acid sequence set forth in SEQ ID NO: 43, and a light chain variable region of the anti-CTLA4 immunoglobulin has the amino acid sequence set forth in SEQ ID NO: 44; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20.

In some embodiments of the present disclosure, provided is the method for treating or preventing a malignant tumor of the vulva, wherein
a heavy chain of the anti-PD-1 antibody has the amino acid sequence set forth in SEQ ID NO: 40, and a light chain of the anti-PD-1 antibody has the amino acid sequence set forth in SEQ ID NO: 24; or
a heavy chain of the anti-PD-1 antibody has the amino acid sequence set forth in SEQ ID NO: 22, and a light chain of the anti-PD-1 antibody has the amino acid sequence set forth in SEQ ID NO: 24.

In some embodiments of the present disclosure, provided is the method for treating or preventing a malignant tumor of the vulva, wherein the first protein functional region is linked to the second protein functional region either directly or via a linker; and/or the heavy chain variable region of the single chain antibody is linked to the light chain variable region of the single chain antibody either directly or via a linker;
preferably, the linker is independently the polypeptide set forth in SEQ ID NO: 47 or a polypeptide formed by concatenating a plurality of (e.g., 2, 3, 4, 5, or 6) polypeptides set forth in SEQ ID NO: 47;
preferably, the linker is independently a polypeptide having one or more glycines linked to the C-terminus of the polypeptide set forth in SEQ ID NO: 47 or a polypeptide formed by concatenating a plurality of (e.g., 2, 3, 4, 5, or 6) polypeptides set forth in SEQ ID NO: 47;
preferably, the first protein functional region and the second protein functional region are independently 1, 2, or more in number.

In some embodiments of the present disclosure, provided is the method for treating or preventing a malignant tumor of the vulva, wherein the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and
the second protein functional region is an anti-CTLA4 single chain antibody;
a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26; preferably, the first linker and the second linker both have the amino acid sequence set forth in SEQ ID NO: 26.

In some embodiments of the present disclosure, provided is the method for treating or preventing a malignant tumor of the vulva, wherein the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody; a heavy chain of the anti-PD-1 immunoglobulin has the amino acid sequence set forth in SEQ ID NO: 22, and a light chain of the anti-PD-1 immunoglobulin has the amino acid sequence set forth in SEQ ID NO: 24;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26; preferably, the first linker and the second linker both have the amino acid sequence set forth in SEQ ID NO: 26.

The present disclosure also relates to a method for treating or preventing a malignant tumor of the vulva, which comprises a step of administering to a subject in need an effective amount of a bispecific antibody or a pharmaceutical composition comprising the bispecific antibody, wherein the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody; a heavy chain of the anti-PD-1 immunoglobulin has the amino acid sequence set forth in SEQ ID NO: 40, and a light chain of the anti-PD-1 immunoglobulin has the amino acid sequence set forth in SEQ ID NO: 24;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26; preferably, the first linker and the second linker both have the amino acid sequence set forth in SEQ ID NO: 26.

In some embodiments of the present disclosure, provided is the method for treating or preventing a malignant tumor of the vulva, wherein the malignant tumor of the vulva is one or more selected from squamous cell carcinoma of the vulva, malignant melanoma of the vulva, basal cell carcinoma of the vulva, verrucous carcinoma of the vulva, Paget's disease of the vulva, sarcoma of the vulva, adenocarcinoma of the vulva, and Bartholin gland carcinoma of the vulva;
preferably, the malignant tumor of the vulva is a recurrent malignant tumor or a metastatic malignant tumor of the vulva;
preferably, the malignant tumor of the vulva is an MSI-H/dMMR malignant tumor of the vulva.

In some embodiments of the present disclosure, provided is the method for treating or preventing a malignant tumor of the vulva, wherein, based on the mass of the bispecific antibody, the pharmaceutical composition has a unit dose of 100 mg-1000 mg, 200 mg-800 mg, 200 mg-500 mg, 300 mg-600 mg, 400 mg-500 mg, or 450 mg.

In some embodiments of the present disclosure, provided is the method for treating or preventing a malignant tumor of the vulva, wherein the pharmaceutical composition further comprises one or more pharmaceutically acceptable auxiliary materials;
preferably, the pharmaceutical composition further comprises one or more antitumor chemotherapeutic drugs in effective amounts.

In some embodiments of the present disclosure, provided is the method for treating or preventing a malignant tumor of the vulva, wherein the pharmaceutical composition is an injection; preferably, the pharmaceutical composition is administered by intravenous drip infusion, intravenous injection, subcutaneous injection, or intraperitoneal injection.

MSI refers to microsatellite instability. Microsatellites are short tandem repeats throughout the human genome, including 10-50 repeats of one, two, or more nucleotides. Microsatellites in certain abnormal cells, such as tumors, are altered in length by insertion or deletion of repeat units as compared to normal cells. Such alteration is referred to as MSI. Based on instability and extent, MSI can be classified as microsatellite instability-high (MSI-H), microsatellite instability-low (MSI-L), and microsatellite stability (MSS). The major cause of MSI is DNA mismatch repair (MMR) deficiency. Human mismatch repair genes (MMR genes) can express corresponding mismatch repair proteins through transcription and translation. Expression absence of any MMR protein may lead to mismatch repair deficiency, and the base pair mismatch will accumulate in the process of DNA replication due to such deficiency, ultimately resulting in MSI. About 15% of colorectal cancers are attributed to the MSI pathway. This was first reported in colorectal cancer, and may also occur in gastric cancer, endometrial cancer, adrenocortical carcinoma, and the like (Baretti M et al., Pharmacol Ther., 2018; 189: 45-62). MSI-H/dMMR characteristics were also found in mesothelioma, sarcoma, adrenocortical carcinoma, malignant melanoma, and ovarian germ cell neoplasm in subsequent studies.

MSI-H and dMMR represent the results of two different assays and are biologically consistent, called MSI-H/dMMR or MSI-high/dMMR, while MSI-L and MSS are phenotypes of proficient mismatch repair (pMMR). The detection of dMMR is to perform an immunohistochemical assay of protein expression for four mismatch genes of MSH2, MLH1, MSH6, and PMS2 based on tumor specimens (including surgical specimens and aspiration specimens). Expression absence of any of the four proteins confirms the dMMR; positive results of all the four proteins indicate pMMR, i.e., a complete mismatch repair function. The detection of MSI is to match the length of the repeated DNA sequences (microsatellite sequences) in tumor cells and somatic cells, and to compare the lengths. When 5 standard loci are detected using PCR based on the American NCI standard, inconsistencies in two or more loci indicate instability, defined as MSI-H, one inconsistent locus indicates MSI-L, and 5 consistent loci indicate MSS. High-throughput sequencing (also referred to as next-generation sequencing, or NGS) can also be used as a method for detecting microsatellite instability. When more microsatellite loci are selected, such as more than 5 loci or additional microsatellite loci, for the PCR assay, inconsistency in ≥30% loci is defined as MSI-H, consistency in all loci is defined as MSS, and inconsistency between 0 and 30% is defined as MSI-L. In one or more embodiments of the present disclosure, provided is the method for treating or preventing a malignant tumor of the vulva, wherein the step of administering to a subject in need an effective amount of the anti-CTLA4-anti-PD-1 bispecific antibody is before or after a surgical treatment and/or before or after radiotherapy.

In one or more embodiments of the present disclosure, provided is the method for treating or preventing a malignant tumor of the vulva, wherein
the anti-CTLA4-anti-PD-1 bispecific antibody is administered at a single dose of 0.1-100 mg, preferably 1-10 mg (e.g., 1 mg, 2 mg, 3 mg, 4 mg, 5 mg, 6 mg, 7 mg, 8 mg, 9 mg, or 10 mg) per kg body weight; alternatively, the anti-CTLA4-anti-PD-1 bispecific antibody is administered at a single dose of 10-1000 mg (e.g., about 100 mg, about 150 mg, about 200 mg, about 250 mg, about 300 mg, about 350 mg, about 400 mg, about 450 mg, about 500 mg, about 600 mg, about 700 mg, about 800 mg, about 900 mg, or about 1000 mg), preferably 50-500 mg, 100-400 mg, 150-300 mg, 150-250 mg, or 200 mg in each subject;
preferably, administration is performed once every 3 days, 4 days, 5 days, 6 days, 10 days, 1 week, 2 weeks, or 3 weeks;
preferably, the route of administration is intravenous drip infusion, intravenous injection, or intraperitoneal injection.

In some embodiments, the administration of the anti-CTLA4-anti-PD-1 bispecific antibody is performed in cycles of 2 weeks (14 days) or 3 weeks (21 days), and preferably, the anti-CTLA4-anti-PD-1 bispecific antibody is administered intravenously on the first day (D1) of each cycle. For example, the anti-CTLA4-anti-PD-1 bispecific antibody is administered once every two weeks (q2w) or three weeks (q3w).

Antibody drugs, especially monoclonal antibodies (mAbs), have achieved good efficacy in the treatment of various diseases. Conventional methods for acquiring these therapeutic antibodies include immunizing animals with an antigen and acquiring antibodies targeting the antigen in the immunized animals, or modifying those antibodies with lower affinity for the antigen by affinity maturation.

The variable regions of the light chain and the heavy chain determine the binding of the antigen; the variable region of each chain comprises three hypervariable regions, i.e., complementarity determining regions (CDRs) (the CDRs of the heavy chain (H) include HCDR1, HCDR2, HCDR3, and the CDRs of the light chain (L) include LCDR1, LCDR2, LCDR3; defined by Kabat et al., see Sequences of Proteins of Immunological Interest, Fifth Edition (1991), Volumes 1-3, NIH Publication 91-3242, Bethesda Md).

The amino acid sequences of the CDRs of the monoclonal antibody sequences in (1)-(13) below are analyzed by technical means well known to those skilled in the art, for example, by a VBASE2 database, and the results are as follows:

### (1) 14C12

The heavy chain variable region has the amino acid sequence set forth in SEQ ID NO: 14, and the light chain variable region has the amino acid sequence set forth in SEQ ID NO: 16.

The amino acid sequences of the 3 CDRs of the heavy chain variable region are as follows:
HCDR1: GFAFSSYD (SEQ ID NO: 27)
HCDR2: ISGGGRYT (SEQ ID NO: 28)
HCDR3: ANRYGEAWFAY (SEQ ID NO: 29)

The amino acid sequences of the 3 CDRs of the light chain variable region are as follows:
LCDR1: QDINTY (SEQ ID NO: 30)
LCDR2: RAN (SEQ ID NO: 31)
LCDR3: LQYDEFPLT (SEQ ID NO: 32)

### (2) 14C12H1L1

The heavy chain variable region has the amino acid sequence set forth in SEQ ID NO: 18, and the light chain variable region has the amino acid sequence set forth in SEQ ID NO: 20.

The amino acid sequences of the 3 CDRs of the heavy chain variable region are identical to those of 14C12.

The amino acid sequences of the 3 CDRs of the light chain variable region are identical to those of 14C12.

### (3) 4G10

The heavy chain variable region has the amino acid sequence set forth in SEQ ID NO: 2, and the light chain variable region has the amino acid sequence set forth in SEQ ID NO: 4.

The amino acid sequences of the 3 CDRs of the heavy chain variable region are as follows:
HCDR1: GYSFTGYT (SEQ ID NO: 33)
HCDR2: INPYNNIT (SEQ ID NO: 34)
HCDR3: ARLDYRSY (SEQ ID NO: 35)

The amino acid sequences of the 3 CDRs of the light chain variable region are as follows:
LCDR1: TGAVTTSNF (SEQ ID NO: 36)
LCDR2: GTN (SEQ ID NO: 37)
LCDR3: ALWYSNHWV (SEQ ID NO: 38)

### (4) 4G10H1L1

The heavy chain variable region has the amino acid sequence set forth in SEQ ID NO: 6, and the light chain variable region has the amino acid sequence set forth in SEQ ID NO: 8.

The amino acid sequences of the 3 CDRs of the heavy chain variable region are identical to those of 4G10.

The amino acid sequences of the 3 CDRs of the light chain variable region are identical to those of 4G10.

### (5) 4G10H3L3

The heavy chain variable region has the amino acid sequence set forth in SEQ ID NO: 10, and the light chain variable region has the amino acid sequence set forth in SEQ ID NO: 12.

The amino acid sequences of the 3 CDRs of the heavy chain variable region are identical to those of 4G10.

The amino acid sequences of the 3 CDRs of the light chain variable region are identical to those of 4G10.

### (6) BiAb001(M)

The amino acid sequences of the 9 CDRs associated with the heavy chain variable region are as follows:
HCDR1: GFAFSSYD (SEQ ID NO: 27)
HCDR2: ISGGGRYT (SEQ ID NO: 28)
HCDR3: ANRYGEAWFAY (SEQ ID NO: 29)
HCDR4: GYSFTGYT (SEQ ID NO: 33)
HCDR5: INPYNNIT (SEQ ID NO: 34)
HCDR6: ARLDYRSY (SEQ ID NO: 35)
HCDR7: TGAVTTSNF (SEQ ID NO: 36)
HCDR8: GTN (SEQ ID NO: 37)
HCDR9: ALWYSNHWV (SEQ ID NO: 38)

The amino acid sequences of the 3 CDRs associated with the light chain variable region are as follows:
LCDR1: QDINTY (SEQ ID NO: 30)
LCDR2: RAN (SEQ ID NO: 31)
LCDR3: LQYDEFPLT (SEQ ID NO: 32)

### (7) BiAb002(M)

The amino acid sequences of the 9 CDRs associated with the heavy chain variable regions are identical to those of BiAb001(M).

The amino acid sequences of the 3 CDRs associated with the light chain variable region are identical to those of BiAb001(M).

### (8) BiAb003(M)

The amino acid sequences of the 9 CDRs associated with the heavy chain variable regions are identical to those of BiAb001(M).

The amino acid sequences of the 3 CDRs associated with the light chain variable region are identical to those of BiAb001(M).

### (9) BiAb004(M)

The amino acid sequences of the 9 CDRs associated with the heavy chain variable regions are identical to those of BiAb001(M).

The amino acid sequences of the 3 CDRs associated with the light chain variable region are identical to those of BiAb001(M).

For the antibody BiAb004(hG1TM) of the present disclosure, amino acid mutations are introduced into the non-variable region of BiAb004(M). According to the EU numbering system, amino acid mutations are introduced at positions 234, 235, and 237:
BiAb004(hG1TM) is obtained by introducing a leucine-to-alanine point mutation at position 234 (L234A), a leucine-to-alanine point mutation at position 235 (L235A), and a glycine-to-alanine point mutation at position 237 (G237A) in the hinge region of the heavy chain.

In the present disclosure, unless otherwise defined, the scientific and technical terms used herein have the meanings generally understood by those skilled in the art. In addition, the laboratory operations of cell culture, molecular genetics, nucleic acid chemistry, and immunology used herein are the routine procedures widely used in the corresponding fields. Meanwhile, in order to better understand the present disclosure, the definitions and explanations of the related terms are provided below.

As used herein, when referring to the amino acid sequence of CTLA4 protein (cytotoxic T-lymphocyte-associated antigen 4), it includes the full length of CTLA4 protein, or the extracellular fragment CTLA4ECD of CTLA4 or a fragment comprising CTLA4ECD, and it also includes a fusion protein of CTLA4ECD, such as a fragment fused to an Fc protein fragment of a mouse or human IgG (mFc or hFc). However, those skilled in the art will appreciate that in the amino acid sequence of the CTLA4 protein, mutations or variations (including but not limited to, substitutions, deletions, and/or additions) can be naturally produced or artificially introduced without affecting biological functions thereof. Therefore, in the present disclosure, the term "CTLA4 protein" shall include all such sequences, including their natural or artificial variants. In addition, when describing a sequence fragment of the CTLA4 protein, it also includes the corresponding sequence fragments in their natural or artificial variants.

As used herein, when referring to the amino acid sequence of PD-1 protein (e.g., NCBI GenBank: NM_005018), it includes the full length of PD-1 protein, or the extracellular fragment PD-1ECD of PD-1 or a fragment comprising PD-1ECD, and it also includes a fusion protein of PD-1ECD, such as a fragment fused to an Fc protein fragment of a mouse or human IgG (mFc or hFc). However, those skilled in the art will appreciate that in the amino acid sequence of the PD-1 protein, mutations or variations (including but not limited to, substitutions, deletions, and/or additions) can be naturally produced or artificially introduced without affecting biological functions thereof. Therefore, in the present disclosure, the term "PD-1 protein" shall include all such sequences, including their natural or artificial variants. In addition, when describing a sequence fragment of the PD-1 protein, it also includes the corresponding sequence fragments in their natural or artificial variants.

As used herein, unless otherwise specified, the B7 is B7-1 and/or B7-2; specific protein sequences thereof are those known in the prior art, and reference may be made to sequences disclosed in the existing literature or GenBank. For example, B7-1 (CD80, NCBI Gene ID: 941); B7-2 (CD86, NCBI Gene ID: 942).

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair consisting of one "light" (L) chain and one "heavy" (H) chain). In a general sense, the heavy chain can be interpreted as a polypeptide chain with a larger molecular weight in an antibody, and the light chain refers to a polypeptide chain with a smaller molecular weight in an antibody. Light chains can be classified into κ and λ light chains. Heavy chains are generally classified into µ, δ, γ, α, and ε, and the antibodies are defined as IgM, IgD, IgG, IgA, and IgE isotypes, respectively. In light chains and heavy chains, the variable region and constant region are linked by a "J" region of about 12 or more amino acids, and the heavy chain also comprises a "D" region of about 3 or more amino acids. Each heavy chain consists of a heavy chain variable region (V_{H}) and a heavy chain constant region (C_{H}). The heavy chain constant region consists of 3 domains (C_{H1}, C_{H2}, and C_{H3}). Each light chain consists of a light chain variable region (V_{L}) and a light chain constant region (C_{L}). The light chain constant region consists of one domain C_{L}. The constant region of the antibody can mediate the binding of immunoglobulins to host tissues or factors, including the binding of various cells of the immune system (e.g., effector cells) to the first component (C1q) of the classical complement system. The V_{H} and V_{L} regions can be further subdivided into hypervariable regions (called complementarity determining regions, or CDRs), and conservative regions called framework regions (FRs) are distributed between the CDRs. Each V_{H} and V_{L} consists of 3 CDRs and 4 FRs arranged from the amino terminus to the carboxyl terminus in the following order: FR1, CDR1, FR2, CDR2, FR3, CDR3, FR4. The variable regions (V_{H} and V_{L}) of each heavy chain/light chain pair form an antibody binding site. The assignment of amino acids to each region or domain follows the definition of Kabat Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883. In particular, the heavy chain may further comprise 3 or more CDRs, such as 6, 9, or 12. For example, in the bispecific antibody of the present disclosure, the heavy chain may be a heavy chain of an IgG antibody with the C-terminus linked to an scFv of another antibody, and in this case, the heavy chain comprises 9 CDRs. The term "antibody" is not limited by any specific method for producing the antibody. For example, the antibody includes, in particular, a recombinant antibody, a monoclonal antibody, and a polyclonal antibody. The antibody may be antibodies of different isotypes, such as IgG (e.g., subtype IgG1, IgG2, IgG3, or IgG4), IgA1, IgA2, IgD, IgE, or IgM.

Antigen-binding fragments (e.g., the above-mentioned antibody fragments) of antibodies can be obtained from given antibodies by using conventional techniques known to those skilled in the art (e.g., recombinant DNA technology, or enzymatic or chemical cleavage), and the antigen-binding fragments of the antibodies are screened for specificity in the same way as for intact antibodies. As used herein, the terms "mAb" and "monoclonal antibody" refer to an antibody or a fragment of an antibody that is derived from a group of highly homologous antibody molecules, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least two or more different antibodies which generally recognize different epitopes on an antigen. Monoclonal antibodies can generally be obtained using hybridoma technique first reported by Kohler et al. (Kohler et al., Nature, 256:495, 1975), but can also be obtained using recombinant DNA technology (see, e.g., U.S. Patent No. 4,816,567).

As used herein, the term "humanized antibody" refers to an antibody or an antibody fragment obtained when all or a part of CDR regions of a human immunoglobulin (receptor antibody) are replaced by the CDR regions of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, for example, Jones et al., Nature, 321:522-525 (1986); Reichmann et al., Nature, 332:323-329 (1988); Presta, Curr. Op. Struct. Biol., 2:593-596 (1992); and Clark, Immunol. Today, 21: 397-402 (2000).

As used herein, the term "antigen-binding fragment" of the antibody, also known as the "antigen-binding portion", refers to a polypeptide comprising the fragment of a full-length antibody, which maintains the ability to specifically bind to the same antigen to which the full-length antibody binds, and/or competes with the full-length antibody for the specific binding to the antigen. See generally, Fundamental Immunology, Ch. 7 (Paul, W., ed., 2nd edition, Raven Press, N.Y. (1989), which is incorporated herein by reference in its entirety for all purposes. The antigen-binding fragment of the antibody can be produced by recombinant DNA technology or by enzymatic or chemical cleavage of an intact antibody. In some cases, the antigen-binding fragment includes Fab, Fab', F(ab')₂, Fd, Fv, dAb, a complementarity determining region (CDR) fragment, a single chain antibody (e.g., scFv), a chimeric antibody, a diabody, and a polypeptide comprising at least a portion of the antibody sufficient to impart specific antigen binding ability to the polypeptide.

As used herein, the term "Fd fragment" refers to an antibody fragment consisting of VH and CH1 domains; the term "Fv fragment" refers to an antibody fragment consisting of the VL and VH domains of a single arm of an antibody; the term "dAb fragment" refers to an antibody fragment consisting of a VH domain (Ward et al., Nature, 341:544-546 (1989)); the term "Fab fragment" refers to an antibody fragment consisting of VL, VH, CL, and CH1 domains; the term "F(ab')₂ fragment" refers to an antibody fragment comprising two Fab fragments linked by a disulfide bridge on a hinge region.

In some cases, the antigen-binding fragment of the antibody is a single chain antibody (e.g., scFv) in which the VL and VH domains are paired to form a monovalent molecule via a linker that enables them to produce a single polypeptide chain (see, e.g., Bird et al., Science, 242:423-426 (1988) and Huston et al., Proc. Natl. Acad. Sci. USA, 85:5879-5883 (1988)). Such scFv molecules may have a general structure: NH2-VL-linker-VH-COOH or NH2-VH-linker-VL-COOH. An appropriate linker in the prior art consists of GGGGS amino acid sequence repeats or a variant thereof. For example, a linker having the amino acid sequence (GGGGS)4 may be used, but a variant thereof may also be used (Holliger et al., (1993), Proc. Natl. Acad. Sci. USA, 90: 6444-6448). Other linkers that may be used in the present disclosure are described by Alfthan et al., (1995), Protein Eng., 8:725-731; Choi et al., (2001), Eur. J. Immunol., 31: 94-106; Hu et al., (1996), Cancer Res., 56:3055-3061; Kipriyanov et al., (1999), J. Mol. Biol., 293:41-56; and Roovers et al., (2001), Cancer Immunol.

As used herein, the term "epitope" refers to a site on the antigen that an immunoglobulin or antibody specifically binds to. The "epitope" is also referred to in the art as an "antigenic determinant". The epitope or antigenic determinant generally consists of chemically active surface groups of molecules such as amino acids, carbohydrates, or sugar side chains, and usually has specific three-dimensional structural characteristics and specific charge characteristics. For example, an epitope typically comprises at least 3, 4, 5, 6, 7, 8, 9, 10, 11, 12, 13, 14, or 15 contiguous or non-contiguous amino acids in a unique spatial conformation, and it may be "linear" or "conformational". See, e.g., Epitope Mapping Protocols in Methods in Molecular Biology, Vol. 66, G. E. Morris, Ed. (1996). In a linear epitope, all interaction sites between a protein and an interaction molecule (e.g., an antibody) are located linearly along the primary amino acid sequence of the protein. In a conformational epitope, the interaction sites are located across amino acid residues of a protein that are separated from each other.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term *"Escherichia coli* expression system" refers to an expression system consisting of *Escherichia coli* (strain) and a vector, wherein the *Escherichia coli* (strain) is from a commercially available strain, such as but not limited to GI698, ER2566, BL21 (DE3), B834 (DE3), and BLR (DE3). As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction, or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as λ phages or M13 phages; and animal viruses. Animal viruses that can be used as vectors include, but are not limited to, retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements, and reporter genes. In addition, the vector may further comprise a replication initiation site.

As used herein, the term "host cell" refers to cells to which vectors can be introduced, including, but not limited to, prokaryotic cells such as *Escherichia coli* or *Bacillus subtilis,* fungal cells such as yeast cells or *Aspergillus,* insect cells such as S2 *Drosophila* cells or Sf9, or animal cells such as fibroblasts, CHO cells, COS cells, NSO cells, HeLa cells, BHK cells, HEK 293 cells, or human cells.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) means that the antibody binds to the antigen with an affinity (K_{D}) of less than about 10⁻⁵ M, e.g., less than about 10⁻⁶ M, 10⁻⁷ M, 10⁻⁸ M, 10⁻⁹ M, or 10⁻¹⁰ M or less. In some embodiments of the present disclosure, the term "target" refers to specific binding.

As used herein, the term "K_{D}" refers to a dissociation equilibrium constant for a specific antibody-antigen interaction and is used to describe the binding affinity between the antibody and the antigen. A smaller dissociation equilibrium constant indicates a stronger antibody-antigen binding and a higher affinity between the antibody and the antigen. Generally, an antibody binds to an antigen with a dissociation equilibrium constant (K_{D}) of less than about 10⁻⁵ M, such as less than about 10⁻⁶ M, 10⁻⁷ M, 10⁻⁸ M, 10⁻⁹ M, or 10⁻¹⁰ M or less, for example, as measured by a BIACORE surface plasmon resonance (SPR) instrument or a Fortebio Octet molecular interaction instrument.

As used herein, the terms "monoclonal antibody" and "mAb" have the same meaning and are used interchangeably; the terms "polyclonal antibody" and "pAb" have the same meaning and are used interchangeably; the terms "polypeptide" and "protein" have the same meaning and are used interchangeably. Besides, in the present disclosure, amino acids are generally represented by single-letter and three-letter abbreviations known in the art. For example, alanine can be represented by A or Ala.

As used herein, the term "pharmaceutically acceptable auxiliary material" refers to a carrier and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), and includes, but is not limited to, pH regulators, surfactants, adjuvants, and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

As used herein, the term "adjuvant" refers to a non-specific immune enhancer, which can enhance the immune response of an organism to antigens or change the type of immune response when delivered into the organism together with the antigens or in advance. There are various adjuvants, including, but not limited to, aluminum adjuvant (e.g., aluminum hydroxide), Freund's adjuvant (e.g., complete Freund's adjuvant and incomplete Freund's adjuvant), *Corynebacterium parvum,* lipopolysaccharide, cytokine, etc. The Freund's adjuvant is the most commonly used adjuvant in animal experiments. The aluminum hydroxide adjuvant is used more frequently in clinical trials.

As used herein, the term "effective amount" refers to an amount sufficient to obtain or at least partially obtain a desired effect. For example, a prophylactically effective amount (e.g., for a disease associated with binding of CTLA4 to B7 or CTLA4 overactivity, such as a tumor) is an amount sufficient to prevent, stop, or delay the onset of the disease (e.g., a disease associated with binding of CTLA4 to B7 or CTLA4 overactivity, such as a tumor); a therapeutically effective amount is an amount sufficient to cure or at least partially stop a disease and its complications in a patient suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight, and gender, the route of administration, and other treatments given concurrently, etc.

A "recurrent" cancer is one that regenerates at the original site or a distant site after response to a previous treatment (e.g., surgery). A "locally recurrent" cancer is one that occurs at the same site as the previously treated cancer after treatment.

A "metastatic" cancer refers to one that spreads from one part of the body (e.g., the lungs) to another.

In the present disclosure, the terms "first" (e.g., first protein functional region or first linker) and "second" (e.g., second protein functional region or second linker) are used for distinguishing or clarity in expression and do not carry typical sequential meanings, unless otherwise specified.

In the present disclosure, "approximately" or "about" refers to that the value or physical quantity defined fluctuates within a range of 10%, 20%, or 30%, unless otherwise specified. For example, approximately 100 minutes or about 100 minutes may be 90 minutes-110 minutes, 80 minutes-120 minutes, or 70 minutes-130 minutes.

### Beneficial Effects of the Present Disclosure

The bispecific antibody of the present disclosure can effectively treat or prevent the malignant tumor of the vulva and has achieved positive effects.

### BRIEF DESCRIPTION OF THE DRAWINGS

FIG. 1 shows the results of assay for the IFN-γ secretion promoted by the anti-CTLA4-anti-PD-1 bispecific antibody in the mixed lymphocyte reaction involving a cell line of squamous cell carcinoma of the vulva.
FIG. 2 shows the results of assay for the IL-2 secretion promoted by the anti-CTLA4-anti-PD-1 bispecific antibody in the mixed lymphocyte reaction involving a cell line of squamous cell carcinoma of the vulva.

### DETAILED DESCRIPTION

The embodiments of the present disclosure will be described in detail below with reference to the examples. Those skilled in the art will appreciate that the following examples are only for illustrating the present disclosure, and should not be construed as limitations to the scope of the present disclosure. Examples where the specific technologies or conditions are not specified are performed according to the technologies or conditions described in the publications of the art (e.g., see, Molecular Cloning: A Laboratory Manual, authored by J. Sambrook et al., and translated by Huang Peitang et al., third edition, Science Press) or according to the package insert. Reagents or instruments used are commercially available conventional products if the manufacturers thereof are not specified.

In the following examples of the present disclosure, the isotype control antibody used, i.e., hIgG4, was an antibody targeting human anti-hen egg lysozyme (HEL), and the variable region sequence of the antibody is from the study reported by Acierno et al., entitled "Affinity maturation increases the stability and plasticity of the Fv domain of anti-protein antibodies" (Acierno et al., J Mol Biol., 2007; 374(1): 130-46). hIgG4 employs an Ig gamma-4 chain C region (ACCESSION: P01861.1) as the heavy chain constant region, with the S228P mutation introduced to enhance stability, and employs an Ig kappa chain C region (ACCESSION: P01834) as the light chain constant region. The hIgG4 is prepared by Akeso Biopharma Inc.
Heavy chain amino acid sequence of isotype control B12hG1 
QVQLVQSGAEVKKPGASVKVSCQASGYRFSNFVIHWVRQAPGQRFE
WMGWINPYNGNKEFSAKFQDRVTFTADTSANTAYMELRSLRSADTAV
YYCARVGPYSWDDSPQDNYYMDVWGKGTTVIVSSASTKGPSVFPLA
PSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHT
CPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEY
KCKVSNKALPAPIEKTISKAKGQPREPQVYTLPPSREEMTKNQVSLTC
LVKGFYPSDIAVEWESNGQPENNYKTTPPVLDSDGSFFLYSKLTVDKS
RWQQGNVFSCSVMHEALHNHYTQKSLSLSPG (SEQ ID NO: 45)
Light chain amino acid sequence of isotype control B12hG1

EIVLTQSPGTLSLSPGERATFSCRSSHSIRSRRVAWYQHKPGQAPRLVIH
GVSNRASGISDRFSGSGSGTDFTLTITRVEPEDFALYYCQVYGASSYTF
GQGTKLERKRTVAAPSVFIFPPSDEQLKSGTASVVCLLNNFYPREAKV

QWKVDNALQSGNSQESVTEQDSKDSTYSLSSTLTLSKADYEKHKVYA
CEVTHQGLSSPVTKSFNRGEC (SEQ ID NO: 46)

The cell line Raji-PDL1 was constructed by Akeso Biopharma Inc. The cell line Raji-PDL1 was produced by viral infection of Raji cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was plenti6.3/V5-PDL1 (PDL1, Genebank ID: NP_054862.1; vector plenti6.3/V5, purchased from Invitrogen, Cat. No. K5315-20).

Anti-PD-1 monoclonal antibody Opdivo (purchased from BMS, Batch No. ABS3203).

Anti-CTLA4 monoclonal antibody Yervoy (purchased from BMS, Batch No. AAT3892).

### Preparation Example 1: Sequence Design of Anti-CTLA4 Antibodies

The amino acid sequences and encoding nucleotide sequences of the heavy and light chains of the anti-CTLA4 antibody 4G10 and its humanized antibodies 4G10H1L1 and 4G10H3L3 are identical to those of 4G10, 4G10H1L1, and 4G10H3L3 in Chinese Patent Publication No. CN 106967172A, respectively.

### (1) Heavy and light chain variable region sequences of 4G10

The nucleotide sequence of the heavy chain variable region: (372 bp)

CAGGTCAAGCTGCAGGAGTCTGGACCTGAGCTGGTGAAGCCTGGA
GCTTCAATGAAGATATCCTGCAAGGCTTCTGGTTACTCATTCACTGG
CTACACCATGAACTGGGTGAAGCAGAGCCATGGAAAGAACCTTGA
ATGGATTGGACTTATTAATCCTTACAATAATATTACTAACTACAACCA
GAAGTTCATGGGCAAGGCCACATTTACTGTAGACAAGTCATCCAGC
ACAGCCTACATGGAACTCCTCAGACTGACATCTGAAGACTCTGGAG
TCTATTTCTGTGCAAGACTCGACTATAGGTCTTATTGGGGCCAAGGG

ACTCTGGTCACTGTCTCTGCAGCCAAAACGACACCCCCATCTGTCT
AT (SEQ ID NO: 1)

The encoded amino acid sequence: (124 aa)

QVKLQESGPELVKPGASMKISCKASGYSFTGYTMNWVKQSHGKNLE
WIGLINPYNNITNYNQKFMGKATFTVDKSSSTAYMELLRLTSEDSGVY
FCARLDYRSYWGQGTLVTVSAAKTTPPSVY (SEQ ID NO: 2)

The nucleotide sequence of the light chain variable region: (378 bp)

CAGGCTGTTGTGACTCAGGAATCTGCACTCACCACATCACCTGGTG
AAACAGTCACACTCACTTGTCGCTCAAGTACTGGGGCTGTTACAAC
TAGTAACTTTGCCAACTGGGTCCAAGAAAAACCAGATCATTTATTC
ACTAGTCTAATAGGTGGTACCAACAACCGAGCTCCAGGTGTTCCTG
CCAGATTCTCAGGCTCCCTGATTGGAGACAAGGCTGCCCTCACCAT
CACAGGGGCACAGACTGAGGATGAGGCAATATATTTCTGTGCTCTAT
GGTACAGCAACCATTGGGTGTTCGGTGGAGGAACCAAACTGACTG
TCCTAGGCCAGCCCAAGTCTTCGCCATCAGTCACCCTGTTTCAAGG
GCAATTCTGC (SEQ ID NO: 3)

The encoded amino acid sequence: (126 aa)

QAVVTQESALTTSPGETVTLTCRSSTGAVTTSNFANWVQEKPDHLFTS
LIGGTNNRAPGVPARFSGSLIGDKAALTITGAQTEDEAIYFCALWYSNH
WVFGGGTKLTVLGQPKSSPSVTLFQGQFC (SEQ ID NO: 4)

### (2) Heavy and light chain variable region sequences of humanized monoclonal antibody 4G10H1L1

The nucleotide sequence of the heavy chain variable region (4G10H1V): (345 bp)

CAGGTGCAGCTGGTGGAGTCTGGGGCCGAGCTGGTGAAGCCCGGC
GCCTCCATGAAGATCTCTTGCAAGGCCAGCGGATACAGTTTCACTG
GCTATACCATGAACTGGGTCAAACAGGCTCCAGGACAGGGACTGG

AGTGGATCGGGCTGATTAATCCTTACAACAACATCACCAACTACAA
CCAGAAGTTCATGGGAAAAGCAACCTTTACAGTGGACAAGAGCAT
TTCCACAGCCTACATGGAACTGAGCCGGCTGACTTCAGACGATAGC
GGGGTCTATTTTTGTGCAAGGCTGGATTATCGCTCTTACTGGGGGCA
GGGAACTCTGGTCACTGTCTCCGCT (SEQ ID NO: 5)

The encoded amino acid sequence: (115 aa)

QVQLVESGAELVKPGASMKISCKASGYSFTGYTMNWVKQAPGQGLE
WIGLINPYNNITNYNQKFMGKATFTVDKSISTAYMELSRLTSDDSGVY
FCARLDYRSYWGQGTLVTVSA (SEQ ID NO: 6)

The nucleotide sequence of the light chain variable region (4G10L1V): (327 bp)

CAGGCTGTCGTCACTCAGGAACCTTCACTGACTGTGAGCCCAGGA
GGAACTGTCACCCTGACATGCGGAAGCTCCACCGGAGCAGTGACC
ACATCCAACTTCGCCAATTGGGTCCAGGAAAAGCCAGGCCAGGCAT
TTCGATCCCTGATCGGAGGCACAAACAATCGGGCTTCTTGGGTGCC
CGCAAGATTCTCAGGAAGCCTGCTGGGGGGAAAAGCCGCTCTGAC
CATTAGTGGCGCTCAGCCTGAGGACGAAGCCGAGTACTTCTGCGCT
CTGTGGTATAGCAACCACTGGGTGTTTGGCGGGGGAACAAAGCTG
ACTGTGCTG (SEQ ID NO: 7)

The encoded amino acid sequence: (109 aa)

QAVVTQEPSLTVSPGGTVTLTCGSSTGAVTTSNFANWVQEKPGQAFRS
LIGGTNNRASWVPARFSGSLLGGKAALTISGAQPEDEAEYFCALWYSN
HWVFGGGTKLTVL (SEQ ID NO: 8)

### (3) Heavy and light chain variable region sequences of humanized monoclonal antibody 4G10H3L3

The nucleotide sequence of the heavy chain variable region (4G10H3V): (345 bp)

CAGGTGCAGCTGGTCGAGTCTGGGGCCGAAGTGAAGAAACCCGGC

GCCTCAGTGAAGGTCAGCTGCAAGGCCAGCGGGTACAGTTTCACT
GGATATACCATGAACTGGGTCCGACAGGCCCCTGGCCAGGGGCTGG
AGTGGATCGGCCTGATTAACCCTTACAACAACATCACTAACTACGC
ACAGAAGTTCCAGGGGAGAGTGACCTTTACAGTGGACACCAGCAT
TTCCACAGCCTACATGGAACTGTCCCGGCTGAGATCTGACGATACA
GGCGTGTACTTCTGCGCTAGGCTGGATTACCGCAGCTATTGGGGAC
AGGGCACACTGGTGACTGTCAGCGCA (SEQ ID NO: 9)

The encoded amino acid sequence (4G10H3V): (115 aa)

QVQLVESGAEVKKPGASVKVSCKASGYSFTGYTMNWVRQAPGQGLE
WIGLINPYNNITNYAQKFQGRVTFTVDTSISTAYMELSRLRSDDTGVYF
CARLDYRSYWGQGTLVTVSA (SEQ ID NO: 10)

The nucleotide sequence of the light chain variable region (4G10L3V): (327 bp)

CAGGCTGTCGTCACTCAGGAACCTTCACTGACCGTGTCTCCTGGCG
GGACTGTCACCCTGACATGCGGCAGCTCCACAGGGGCCGTGACCA
CAAGTAACTTCCCAAATTGGGTCCAGCAGAAGCCAGGACAGGCTC
CCCGGAGTCTGATCGGAGGCACCAACAACAAGGCCAGCTGGACAC
CCGCACGGTTCAGCGGCAGCCTGCTGGGCGGCAAGGCCGCTCTGA
CAATTAGCGGAGCCCAGCCTGAGGACGAAGCCGAGTACTATTGCGC
TCTGTGGTACTCCAACCACTGGGTGTTCGGCGGCGGCACCAAGCTG
ACTGTGCTG (SEQ ID NO: 11)

The encoded amino acid sequence (4G10L3V): (109 aa)

QAVVTQEPSLTVSPGGTVTLTCGSSTGAVTTSNFPNWVQQKPGQAPRS
LIGGTNNKASWTPARFSGSLLGGKAALTISGAQPEDEAEYYCALWYS
NHWVFGGGTKLTVL (SEQ ID NO: 12)

### Preparation Example 2: Sequence Design of Anti-PD-1 Antibody 14C12 and Its Humanized Antibody 14C12H1L1

The amino acid sequences and encoding nucleotide sequences of the heavy and light chains of the anti-PD-1 antibody 14C12 and its humanized antibody 14C12H1L1 are identical to those of 14C12 and 14C12H1L1 in Chinese Patent Publication No. CN 106967172A, respectively.

### (1) Heavy and light chain variable region sequences of 14C12

The nucleotide sequence of the heavy chain variable region: (354 bp)

GAGGTCAAACTGGTGGAGAGCGGCGGCGGGCTGGTGAAGCCCGG
CGGGTCACTGAAACTGAGCTGCGCCGCTTCCGGCTTCGCCTTTAGC
TCCTACGACATGTCATGGGTGAGGCAGACCCCTGAGAAGCGCCTGG
AATGGGTCGCTACTATCAGCGGAGGCGGGCGATACACCTACTATCCT
GACTCTGTCAAAGGGAGATTCACAATTAGTCGGGATAACGCCAGAA
ATACTCTGTATCTGCAGATGTCTAGTCTGCGGTCCGAGGATACAGCT
CTGTACTATTGTGCAAACCGGTACGGCGAAGCATGGTTTGCCTATTG
GGGACAGGGCACCCTGGTGACAGTCTCTGCC (SEQ ID NO: 13)

The encoded amino acid sequence: (118 aa)

EVKLVESGGGLVKPGGSLKLSCAASGFAFSSYDMSWVRQTPEKRLEW
VATISGGGRYTYYPDSVKGRFTISRDNARNTLYLQMSSLRSEDTALYY
CANRYGEAWFAYWGQGTLVTVSA (SEQ ID NO: 14)

The nucleotide sequence of the light chain variable region: (321 bp)

GACATTAAGATGACACAGTCCCCTTCCTCAATGTACGCTAGCCTGG
GCGAGCGAGTGACCTTCACATGCAAAGCATCCCAGGACATCAACA
CATACCTGTCTTGGTTTCAGCAGAAGCCAGGCAAAAGCCCCAAGAC
CCTGATCTACCGGGCCAATAGACTGGTGGACGGGGTCCCCAGCAGA
TTCTCCGGATCTGGCAGTGGGCAGGATTACTCCCTGACCATCAGCTC
CCTGGAGTATGAAGACATGGGCATCTACTATTGCCTGCAGTATGATG
AGTTCCCTCTGACCTTTGGAGCAGGCACAAAACTGGAACTGAAG
(SEQ ID NO: 15)

The encoded amino acid sequence: (107 aa)

DIKMTQSPSSMYASLGERVTFTCKASQDINTYLSWFQQKPGKSPKTLI

YRANRLVDGVPSRFSGSGSGQDYSLTISSLEYEDMGIYYCLQYDEFPLT
FGAGTKLELK (SEQ ID NO: 16)

### (2) Heavy and light chain variable region sequences and heavy and light chain sequences of humanized monoclonal antibody 14C12H1L1

The nucleotide sequence of the heavy chain variable region: (354 bp)

GAAGTGCAGCTGGTCGAGTCTGGGGGAGGGCTGGTGCAGCCCGGC
GGGTCACTGCGACTGAGCTGCGCAGCTTCCGGATTCGCCTTTAGCT
CCTACGACATGTCCTGGGTGCGACAGGCACCAGGAAAGGGACTGG
ATTGGGTCGCTACTATCTCAGGAGGCGGGAGATACACCTACTATCCT
GACAGCGTCAAGGGCCGGTTCACAATCTCTAGAGATAACAGTAAGA
ACAATCTGTATCTGCAGATGAACAGCCTGAGGGCTGAGGACACCGC
ACTGTACTATTGTGCCAACCGCTACGGGGAAGCATGGTTTGCCTATT
GGGGGCAGGGAACCCTGGTGACAGTCTCTAGT (SEQ ID NO: 17)

The encoded amino acid sequence: (118 aa)

EVQLVESGGGLVQPGGSLRLSCAASGFAFSSYDMSWVRQAPGKGLD
WVATISGGGRYTYYPDSVKGRFTISRDNSKNNLYLQMNSLRAEDTALY
YCANRYGEAWFAYWGQGTLVTVSS (SEQ ID NO: 18)

The nucleotide sequence of the light chain variable region: (321 bp)

GACATTCAGATGACTCAGAGCCCCTCCTCCATGTCCGCCTCTGTGG
GCGACAGGGTCACCTTCACATGCCGCGCTAGTCAGGATATCAACAC
CTACCTGAGCTGGTTTCAGCAGAAGCCAGGGAAAAGCCCCAAGAC
ACTGATCTACCGGGCTAATAGACTGGTGTCTGGAGTCCCAAGTCGG
TTCAGTGGCTCAGGGAGCGGACAGGACTACACTCTGACCATCAGCT
CCCTGCAGCCTGAGGACATGGCAACCTACTATTGCCTGCAGTATGAT
GAGTTCCCACTGACCTTTGGCGCCGGGACAAAACTGGAGCTGAAG
(SEQ ID NO: 19)

The encoded amino acid sequence: (107 aa)

DIQMTQSPSSMSASVGDRVTFTCRASQDINTYLSWFQQKPGKSPKTLI
YRANRLVSGVPSRFSGSGSGQDYTLTISSLQPEDMATYYCLQYDEFPLT
FGAGTKLELK (SEQ ID NO: 20)

The DNA sequence of the 14C12H1L1 heavy chain (14C12H1): (1344 bp)

GAAGTGCAGCTGGTCGAGTCTGGGGGAGGGCTGGTGCAGCCCGGC
GGGTCACTGCGACTGAGCTGCGCAGCTTCCGGATTCGCCTTTAGCT
CCTACGACATGTCCTGGGTGCGACAGGCACCAGGAAAGGGACTGG
ATTGGGTCGCTACTATCTCAGGAGGCGGGAGATACACCTACTATCCT
GACAGCGTCAAGGGCCGGTTCACAATCTCTAGAGATAACAGTAAGA
ACAATCTGTATCTGCAGATGAACAGCCTGAGGGCTGAGGACACCGC
ACTGTACTATTGTGCCAACCGCTACGGGGAAGCATGGTTTGCCTATT
GGGGGCAGGGAACCCTGGTGACAGTCTCTAGTGCCAGCACCAAAG
GACCTAGCGTGTTTCCTCTCGCCCCCTCCTCCAAAAGCACCAGCGG
AGGAACCGCTGCTCTCGGATGTCTGGTGAAGGACTACTTCCCTGAA
CCCGTCACCGTGAGCTGGAATAGCGGCGCTCTGACAAGCGGAGTC
CATACATTCCCTGCTGTGCTGCAAAGCAGCGGACTCTATTCCCTGTC
CAGCGTCGTCACAGTGCCCAGCAGCAGCCTGGGCACCCAGACCTA
CATCTGTAACGTCAACCACAAGCCCTCCAACACCAAGGTGGACAA
GAAAGTGGAGCCCAAATCCTGCGACAAGACACACACCTGTCCCCC
CTGTCCTGCTCCCGAACTCCTCGGAGGCCCTAGCGTCTTCCTCTTTC
CTCCCAAACCCAAGGACACCCTCATGATCAGCAGAACCCCTGAAGT
CACCTGTGTCGTCGTGGATGTCAGCCATGAGGACCCCGAGGTGAAA
TTCAACTGGTATGTCGATGGCGTCGAGGTGCACAACGCCAAAACCA
AGCCCAGGGAGGAACAGTACAACTCCACCTACAGGGTGGTGTCCG
TGCTGACAGTCCTCCACCAGGACTGGCTGAACGGCAAGGAGTACA
AGTGCAAGGTGTCCAACAAGGCTCTCCCTGCCCCCATTGAGAAGA
CCATCAGCAAGGCCAAAGGCCAACCCAGGGAGCCCCAGGTCTATA
CACTGCCTCCCTCCAGGGACGAACTCACCAAGAACCAGGTGTCCC

TGACCTGCCTGGTCAAGGGCTTTTATCCCAGCGACATCGCCGTCGA
GTGGGAGTCCAACGGACAGCCCGAGAATAACTACAAGACCACCCC
TCCTGTCCTCGACTCCGACGGCTCCTTCTTCCTGTACAGCAAGCTG
ACCGTGGACAAAAGCAGGTGGCAGCAGGGAAACGTGTTCTCCTGC
AGCGTGATGCACGAAGCCCTCCACAACCACTACACCCAGAAAAGC
CTGTCCCTGAGCCCCGGCAAA (SEQ ID NO: 21)

The encoded amino acid sequence: (448 aa)

EVQLVESGGGLVQPGGSLRLSCAASGFAFSSYDMSWVRQAPGKGLD
WVATISGGGRYTYYPDSVKGRFTISRDNSKNNLYLQMNSLRAEDTALY
YCANRYGEAWFAYWGQGTLVTVSSASTKGPSVFPLAPSSKSTSGGTA
ALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPELLG
GPSVFLFPPKPKDTLMISRTPEVTCVVVDVSHEDPEVKFNWYVDGVE
VHNAKTKPREEQYNSTYRVVSVLTVLHQDWLNGKEYKCKVSNKALP
APIEKTISKAKGQPREPQVYTLPPSRDELTKNQVSLTCLVKGFYPSDIAV
EWESNGQPENNYKTTPPVLDSDGSFFLYSKLTVDKSRWQQGNVFSCS
VMHEALHNHYTQKSLSLSPGK (SEQ ID NO: 22)

The DNA sequence of the 14C12H1L1 light chain (14C12L1): (642 bp)

GACATTCAGATGACTCAGAGCCCCTCCTCCATGTCCGCCTCTGTGG
GCGACAGGGTCACCTTCACATGCCGCGCTAGTCAGGATATCAACAC
CTACCTGAGCTGGTTTCAGCAGAAGCCAGGGAAAAGCCCCAAGAC
ACTGATCTACCGGGCTAATAGACTGGTGTCTGGAGTCCCAAGTCGG
TTCAGTGGCTCAGGGAGCGGACAGGACTACACTCTGACCATCAGCT
CCCTGCAGCCTGAGGACATGGCAACCTACTATTGCCTGCAGTATGAT
GAGTTCCCACTGACCTTTGGCGCCGGGACAAAACTGGAGCTGAAG
CGAACTGTGGCCGCTCCCTCCGTCTTCATTTTTCCCCCTTCTGACGA
ACAGCTGAAATCAGGCACAGCCAGCGTGGTCTGTCTGCTGAACAAT
TTCTACCCTAGAGAGGCAAAAGTGCAGTGGAAGGTCGATAACGCC

CTGCAGTCCGGCAACAGCCAGGAGAGTGTGACTGAACAGGACTCA
AAAGATAGCACCTATTCCCTGTCTAGTACACTGACTCTGTCCAAGGC
TGATTACGAGAAGCACAAAGTGTATGCATGCGAAGTGACACATCAG
GGACTGTCAAGCCCCGTGACTAAGTCTTTTAACCGGGGCGAATGT
(SEQ ID NO: 23)

The encoded amino acid sequence: (214 aa)

DIQMTQSPSSMSASVGDRVTFTCRASQDINTYLSWFQQKPGKSPKTLI
YRANRLVSGVPSRFSGSGSGQDYTLTISSLQPEDMATYYCLQYDEFPLT
FGAGTKLELKRTVAAPSVFIFPPSDEQLKSGTASVVCLLNNFYPREAKV
QWKVDNALQSGNSQESVTEQDSKDSTYSLSSTLTLSKADYEKHKVYA
CEVTHQGLSSPVTKSFNRGEC (SEQ ID NO: 24)

### Preparation Example 3: Sequence Design of Bispecific Antibodies BiAb001(M). BiAb002(M). BiAb003(M), and BiAb004(M)

The structural patterns of the bispecific antibodies BiAb001(M), BiAb002(M), BiAb003(M), and BiAb004(M) are in the Morrison format (IgG-scFv), i.e., the C-termini of two heavy chains of one IgG antibody are linked to the scFv fragments of another antibody, respectively, via linkers. The components for the heavy and light chain design are shown in Table A below.

**Table A: Sequence design of BiAb001(M), BiAb002(M), BiAb003(M), and BiAb004(M)**

| Bispecific antibody | Immunoglobulin portion | | Linker | scFv portion |
|---|---|---|---|---|
| | Heavy chain | Light chain | | |
| BiAb001(M) | 14C12H1 | 14C12L1 | Linker1 | 4G10H1V(M)-Linker2 - 4G10L1V(M) |
| BiAb002(M) | 14C12H1 | 14C12L1 | Linker2 | 4G10H1V(M)-Linker2 -4G10L1V(M) |
| BiAb003(M) | 14C12H1 | 14C12L1 | Linker1 | 4G10H3V(M)-Linker2 - 4G10L3V(M) |
| BiAb004(M) | 14C12H1 | 14C12L1 | Linker2 | 4G10H3V(M)-Linker2 - 4G10L3V(M) |

In Table A above:
Amino acid sequence of Linker1 : GGGGSGGGGSGGGGS (SEQ ID NO: 25)
Amino acid sequence of Linker2: GGGGSGGGGSGGGGSGGGGS (SEQ ID NO: 26)

Additionally, in Table A above, 4G10H1V(M), 4G10L1V(M), 4G10H3V(M), and 4G10L3V(M) in the scFv fragments of antibodies BiAb001(M), BiAb002(M), BiAb003(M), and BiAb004(M) are based on 4G10H1V, 4G10L1V, 4G10H3V, and 4G10L3V, respectively, with individual amino acids in the framework regions mutated, and this effectively optimizes the structure of the antibodies and improves their efficacy.
(1) 4G10H1V(M): (115 aa, mutation site underlined in the amino acid sequence based on 4G10H1V) 
QVQLVESGAELVKPGASMKISCKASGYSFTGYTMNWVKQAPGQCLE
WIGLINPYNNITNYNQKFMGKATFTVDKSISTAYMELSRLTSDDSGVY
FCARLDYRSYWGQGTLVTVSA (SEQ ID NO: 41)
(2) 4G10L1V(M): (110 aa, mutation sites underlined in the amino acid sequence based on 4G10L1V) 
QAVVTQEPSLTVSPGGTVTLTCGSSTGAVTTSNFANWVQEKPGQAFRS
LIGGTNNRASWVPARFSGSLLGGKAALTISGAQPEDEAEYFCALWYSN
HWVFGCGTKLTVLR (SEQ ID NO: 42)
(3) 4G10H3V(M): (115 aa, mutation site underlined in the amino acid sequence based on 4G10H3V) 
QVQLVESGAEVKKPGASVKVSCKASGYSFTGYTMNWVRQAPGQCLE
WIGLINPYNNITNYAQKFQGRVTFTVDTSISTAYMELSRLRSDDTGVYF
 CARLDYRSYWGQGTLVTVSA (SEQ ID NO: 43)
(4) 4G10L3V(M): (110 aa, mutation sites underlined in the amino acid sequence based on 4G10L3V)

QAVVTQEPSLTVSPGGTVTLTCGSSTGAVTTSNFPNWVQQKPGQAPRS
LIGGTNNKASWTPARFSGSLLGGKAALTISGAQPEDEAEYYCALWYS
NHWVFGCGTKLTVLR (SEQ ID NO: 44)

In order to distinguish it from the mutant antibodies hereinafter, BiAb004(M) is also referred to as BiAb004(hG1WT) in the examples of the present disclosure. BiAb004(M) described above is the "wild-type", comprising an Ig gamma-1 chain C region (ACCESSION: P01857) as the heavy chain constant region and an Ig kappa chain C region (ACCESSION: P01834) as the light chain constant region.

### Preparation Example 4: Non-Variable Region Amino Acid Mutation Design Based on Humanized Bispecific Antibody BiAb004

On the basis of BiAb004(hG1WT) obtained in Preparation Example 3, BiAb004(hG1TM) was obtained by introducing a leucine-to-alanine point mutation at position 234 (L234A), a leucine-to-alanine point mutation at position 235 (L235A), and a glycine-to-alanine point mutation at position 237 (G237A) in the heavy chain by the inventors.

The DNA sequence of the heavy chain of the immunoglobulin portion in BiAb004(hG1TM): (1344 bp, mutation sites underlined)

GAAGTGCAGCTGGTCGAGTCTGGGGGAGGGCTGGTGCAGCCCGGC
GGGTCACTGCGACTGAGCTGCGCAGCTTCCGGATTCGCCTTTAGCT
CCTACGACATGTCCTGGGTGCGACAGGCACCAGGAAAGGGACTGG
ATTGGGTCGCTACTATCTCAGGAGGCGGGAGATACACCTACTATCCT
GACAGCGTCAAGGGCCGGTTCACAATCTCTAGAGATAACAGTAAGA
ACAATCTGTATCTGCAGATGAACAGCCTGAGGGCTGAGGACACCGC

ACTGTACTATTGTGCCAACCGCTACGGGGAAGCATGGTTTGCCTATT
GGGGGCAGGGAACCCTGGTGACAGTCTCTAGTGCCAGCACCAAAG
GGCCCAGCGTGTTTCCTCTCGCCCCCTCCTCCAAAAGCACCAGCGG
AGGAACCGCTGCTCTCGGATGTCTGGTGAAGGACTACTTCCCTGAA
CCCGTCACCGTGAGCTGGAATAGCGGCGCTCTGACAAGCGGAGTC
CATACATTCCCTGCTGTGCTGCAAAGCAGCGGACTCTATTCCCTGTC
CAGCGTCGTCACAGTGCCCAGCAGCAGCCTGGGCACCCAGACCTA
CATCTGTAACGTCAACCACAAGCCCTCCAACACCAAGGTGGACAA
GAAAGTGGAGCCCAAATCCTGCGACAAGACACACACCTGTCCCCC
CTGTCCTGCTCCCGAAGCTGCTGGAGCCCCTAGCGTCTTCCTCTTTC
CTCCCAAACCCAAGGACACCCTCATGATCAGCAGAACCCCTGAAGT
CACCTGTGTCGTCGTGGATGTCAGCCATGAGGACCCCGAGGTGAAA
TTCAACTGGTATGTCGATGGCGTCGAGGTGCACAACGCCAAAACCA
AGCCCAGGGAGGAACAGTACAACTCCACCTACAGGGTGGTGTCCG
TGCTGACAGTCCTCCACCAGGACTGGCTGAACGGCAAGGAGTACA
AGTGCAAGGTGTCCAACAAGGCTCTCCCTGCCCCCATTGAGAAGA
CCATCAGCAAGGCCAAAGGCCAACCCAGGGAGCCCCAGGTCTATA
CACTGCCTCCCTCCAGGGACGAACTCACCAAGAACCAGGTGTCCC
TGACCTGCCTGGTCAAGGGCTTTTATCCCAGCGACATCGCCGTCGA
GTGGGAGTCCAACGGACAGCCCGAGAATAACTACAAGACCACCCC
TCCTGTCCTCGACTCCGACGGCTCCTTCTTCCTGTACAGCAAGCTG
ACCGTGGACAAAAGCAGGTGGCAGCAGGGAAACGTGTTCTCCTGC
AGCGTGATGCACGAAGCCCTCCACAACCACTACACCCAGAAAAGC
CTGTCCCTGAGCCCCGGCAAA (SEQ ID NO: 39)

The amino acid sequence of the heavy chain of the immunoglobulin portion in BiAb004(hG1TM): (448 aa, mutation sites underlined)

EVQLVESGGGLVQPGGSLRLSCAASGFAFSSYDMSWVRQAPGKGLD
WVATISGGGRYTYYPDSVKGRFTISRDNSKNNLYLQMNSLRAEDTALY

YCANRYGEAWFAYWGQGTLVTVSSASTKGPSVFPLAPSSKSTSGGTA
ALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPEAAG
APSVFLFPPKPKDTLMISRTPEVTCVVVDVSHEDPEVKFNWYVDGVE
VHNAKTKPREEQYNSTYRVVSVLTVLHQDWLNGKEYKCKVSNKALP
APIEKTISKAKGQPREPQVYTLPPSRDELTKNQVSLTCLVKGFYPSDIAV
EWESNGQPENNYKTTPPVLDSDGSFFLYSKLTVDKSRWQQGNVFSCS
VMHEALHNHYTQKSLSLSPGK (SEQ ID NO: 40)

BiAb004(hG1TM) and BiAb004(hG1WT) share the same DNA sequence of the light chain and the same encoded amino acid sequence. The specific sequence is shown in Preparation Example 3.

### Example 1: Biological Activity of Anti-CTLA4-Anti-PD-1 Bispecific Antibody in Promoting IFN-γ and IL-2 Secretion in Mixed Lymphocyte Reaction (MLR) System Involving Cell Line of Squamous Cell Carcinoma of Vulva

Normal human PBMCs (from a healthy donor) were obtained by separation according to the instructions of the Ficoll-Paque^{™} Plus separation solution and cryopreserved. Two days before the experiment, PBMCs were thawed and cultured in a 1640 complete medium in a 5% carbon dioxide incubator at 37 °C. After 2 h, when the state of PBMCs was recovered, SEB (purchased from Toxin technology, Cat. No. BT202, at a final concentration of 0.5 µg/mL) was added for stimulation for two days.

On the day of the experiment, Raji-PDL1 cells (constructed by Akeso Biopharma Inc.) were conventionally collected and centrifuged at 110× g for 5 min, followed by removal of the supernatant. The cells were resuspended with an assay medium (i.e., 1640 + 10% FBS) and counted, and MMC (Mito-mycin C, purchased from Stressmarq, Cat. No. SIH-246-10MG, at a final concentration of 2 µg/mL) was added. The cells were treated in a 5% carbon dioxide incubator at 37 °C for 1 h.

Vulvar cancer cells SW954 (purchased from ATCC, Cat. No. HTB-117) were conventionally collected, centrifuged at 170× g for 5 min, resuspended with an assay medium, and counted, and the cell density was adjusted. PBMCs stimulated with SEB for two days and Raji-PDL1 cells treated with MMC were conventionally collected, washed twice and resuspended with an assay medium, and counted. PBMCs, Raji-PDL1, and SW954 were seeded into 96-well U-shaped plates at 100000 cells/well, 100000 cells/well, and 20000 cells/well, respectively. Experimental grouping was as follows:
BiAb004(hG1TM),
combination of Opdivo + Yervoy,
Raji-PDL1 + PBMC control,
negative control (no antibody added),
30 nM isotype control B12hG1, and
30 nM isotype control hIgG4.

The groups, except for the Raji-PDL1 + PBMC control group, contained 3 types of cells: Raji-PDL1, PBMC, and SW954.

The antibodies were added according to the experimental design, and the final concentrations of the antibody BiAb004(hG1TM) group and the Opdivo + Yervoy group were 30 nM, 10 nM, 3 nM, and 0.3 nM, and the antibodies were co-cultured with the cells for 3 days. After 3 days, the mixtures were centrifuged at 250× g for 5 min (Beckman centrifuge), and the culture supernatants were collected and assayed for IFN-γ and IL-2 contents using a Dakewe kit.

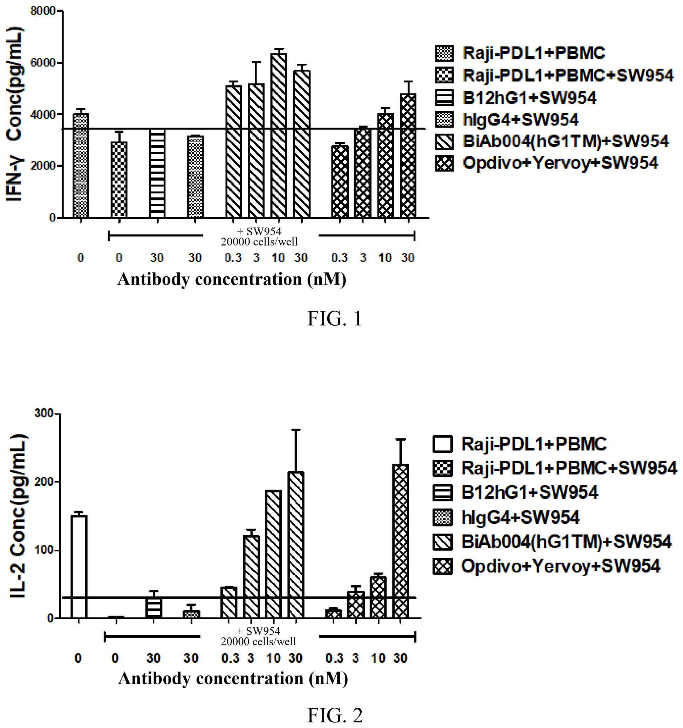

The results are shown in FIG. 1 and FIG. 2. The results show that in the mixed lymphocyte system involving SW954 cells of squamous cell carcinoma of the vulva, both the anti-CTLA4-anti-PD-1 bispecific antibody BiAb004(hG1TM) group and the Opdivo + Yervoy combination group could effectively promote the expression of IFN-γ and IL-2 in the system, and the BiAb004(hG1TM) group had a stronger activity in promoting IFN-γ and IL-2 secretion than the anti-PD-1 monoclonal antibody Opdivo + anti-CTLA4 monoclonal antibody Yervoy combination group.

Although the specific embodiments of the present disclosure have been described in detail, those skilled in the art will appreciate that various modifications and substitutions can be made to those details according to all the teachings that have been disclosed, and these changes shall all fall within the protection scope of the present disclosure. The full scope of the present disclosure is given by the appended claims and any equivalent thereof.

## Claims

1. Use of a bispecific antibody or a pharmaceutical composition comprising the bispecific antibody in the preparation of a medicament for treating or preventing a malignant tumor of vulva, wherein the bispecific antibody comprises:
a first protein functional region targeting PD-1 and
a second protein functional region targeting CTLA4,
wherein:
the first protein functional region is an anti-PD-1 antibody or an antigen-binding fragment thereof, and the anti-PD-1 antibody comprises a heavy chain variable region and a light chain variable region, wherein the heavy chain variable region comprises HCDR1 having the amino acid sequence set forth in SEQ ID NO: 27, HCDR2 having the amino acid sequence set forth in SEQ ID NO: 28, and HCDR3 having the amino acid sequence set forth in SEQ ID NO: 29; and the light chain variable region comprises LCDR1 having the amino acid sequence set forth in SEQ ID NO: 30, LCDR2 having the amino acid sequence set forth in SEQ ID NO: 31, and LCDR3 having the amino acid sequence set forth in SEQ ID NO: 32;
the second protein functional region is an anti-CTLA4 antibody or an antigen-binding fragment thereof, and the anti-CTLA4 antibody comprises a heavy chain variable region and a light chain variable region, wherein the heavy chain variable region comprises HCDR1 having the amino acid sequence set forth in SEQ ID NO: 33, HCDR2 having the amino acid sequence set forth in SEQ ID NO: 34, and HCDR3 having the amino acid sequence set forth in SEQ ID NO: 35; and the light chain variable region comprises LCDR1 having the amino acid sequence set forth in SEQ ID NO: 36, LCDR2 having the amino acid sequence set forth in SEQ ID NO: 37, and LCDR3 having the amino acid sequence set forth in SEQ ID NO: 38.

2. The use according to claim 1, wherein
the heavy chain variable region of the anti-PD-1 antibody has an amino acid sequence selected from SEQ ID NO: 14 and SEQ ID NO: 18, and the light chain variable region of the anti-PD-1 antibody has an amino acid sequence selected from SEQ ID NO: 16 and SEQ ID NO: 20;
preferably, the heavy chain variable region of the anti-PD-1 antibody has the amino acid sequence set forth in SEQ ID NO: 14, and the light chain variable region of the anti-PD-1 antibody has the amino acid sequence set forth in SEQ ID NO: 16; or
preferably, the heavy chain variable region of the anti-PD-1 antibody has the amino acid sequence set forth in SEQ ID NO: 18, and the light chain variable region of the anti-PD-1 antibody has the amino acid sequence set forth in SEQ ID NO: 20.

3. The use according to any one of claims 1 to 2, wherein
the heavy chain variable region of the anti-CTLA4 antibody has an amino acid sequence selected from SEQ ID NO: 2, SEQ ID NO: 6, SEQ ID NO: 10, SEQ ID NO: 41, and SEQ ID NO: 43, and the light chain variable region of the anti-CTLA4 antibody has an amino acid sequence selected from SEQ ID NO: 4, SEQ ID NO: 8, SEQ ID NO: 12, SEQ ID NO: 42, and SEQ ID NO: 44; preferably:
the heavy chain variable region of the anti-CTLA4 antibody has the amino acid sequence set forth in SEQ ID NO: 2, and the light chain variable region of the anti-CTLA4 antibody has the amino acid sequence set forth in SEQ ID NO: 4;
the heavy chain variable region of the anti-CTLA4 antibody has the amino acid sequence set forth in SEQ ID NO: 6, and the light chain variable region of the anti-CTLA4 antibody has the amino acid sequence set forth in SEQ ID NO: 8;
the heavy chain variable region of the anti-CTLA4 antibody has the amino acid sequence set forth in SEQ ID NO: 10, and the light chain variable region of the anti-CTLA4 antibody has the amino acid sequence set forth in SEQ ID NO: 12;
the heavy chain variable region of the anti-CTLA4 antibody has the amino acid sequence set forth in SEQ ID NO: 41, and the light chain variable region of the anti-CTLA4 antibody has the amino acid sequence set forth in SEQ ID NO: 42; or
the heavy chain variable region of the anti-CTLA4 antibody has the amino acid sequence set forth in SEQ ID NO: 43, and the light chain variable region of the anti-CTLA4 antibody has the amino acid sequence set forth in SEQ ID NO: 44.

4. The use according to any one of claims 1 to 3, wherein
the first protein functional region is an anti-PD-1 immunoglobulin, and the second protein functional region is an anti-CTLA4 single chain antibody; preferably, the anti-CTLA4 single chain antibody is linked to the N-terminus or the C-terminus of a heavy chain of the anti-PD-1 immunoglobulin; preferably, the anti-PD-1 immunoglobulin is of human IgG1 subtype; preferably, according to the EU numbering system, a heavy chain constant region of the anti-PD-1 immunoglobulin has one of the following mutation combinations:
L234A and L235A;
L234A and G237A;
L235A and G237A; or
L234A, L235A, and G237A.

5. The use according to any one of claims 1 to 3, wherein
the first protein functional region is an anti-PD-1 single chain antibody, and the second protein functional region is an anti-CTLA4 immunoglobulin;
preferably, the anti-PD-1 single chain antibody is linked to the N-terminus or the C-terminus of a heavy chain of the anti-CTLA4 immunoglobulin;
preferably, the anti-CTLA4 immunoglobulin is of human IgG1 subtype;
preferably, according to the EU numbering system, a heavy chain constant region of the immunoglobulin has one of the following mutation combinations:
L234A and L235A;
L234A and G237A;
L235A and G237A; or
L234A, L235A, and G237A.

6. The use according to any one of claims 1 to 5, wherein
a heavy chain variable region of the anti-PD-1 immunoglobulin has an amino acid sequence selected from SEQ ID NO: 14 and SEQ ID NO: 18, and a light chain variable region of the anti-PD-1 immunoglobulin has an amino acid sequence selected from SEQ ID NO: 16 and SEQ ID NO: 20; and, a heavy chain variable region of the anti-CTLA4 single chain antibody has an amino acid sequence selected from SEQ ID NO: 2, SEQ ID NO: 6, SEQ ID NO: 10, SEQ ID NO: 41, and SEQ ID NO: 43, and a light chain variable region of the anti-CTLA4 single chain antibody has an amino acid sequence selected from SEQ ID NO: 4, SEQ ID NO: 8, SEQ ID NO: 12, SEQ ID NO: 42, and SEQ ID NO: 44;
or
a heavy chain variable region of the anti-CTLA4 immunoglobulin has an amino acid sequence selected from SEQ ID NO: 2, SEQ ID NO: 6, SEQ ID NO: 10, SEQ ID NO: 41, and SEQ ID NO: 43, and a light chain variable region of the anti-CTLA4 immunoglobulin has an amino acid sequence selected from SEQ ID NO: 4, SEQ ID NO: 8, SEQ ID NO: 12, SEQ ID NO: 42, and SEQ ID NO: 44; and, a heavy chain variable region of the anti-PD-1 single chain antibody has an amino acid sequence selected from SEQ ID NO: 14 and SEQ ID NO: 18, and a light chain variable region of the anti-PD-1 single chain antibody has an amino acid sequence selected from SEQ ID NO: 16 and SEQ ID NO: 20;
preferably, the bispecific antibody is selected from any one of the following (1)-(20):
(1) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 2, and a light chain variable region of the anti-CTLA4 single chain antibody has the amino acid sequence set forth in SEQ ID NO: 4;
(2) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 6, and a light chain variable region of the anti-CTLA4 single chain antibody has the amino acid sequence set forth in SEQ ID NO: 8;
(3) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 10, and a light chain variable region of the anti-CTLA4 single chain antibody has the amino acid sequence set forth in SEQ ID NO: 12;
(4) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 2, and a light chain variable region of the anti-CTLA4 single chain antibody has the amino acid sequence set forth in SEQ ID NO: 4;
(5) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 6, and a light chain variable region of the anti-CTLA4 single chain antibody has the amino acid sequence set forth in SEQ ID NO: 8;
(6) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 10, and a light chain variable region of the anti-CTLA4 single chain antibody has the amino acid sequence set forth in SEQ ID NO: 12;
(7) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
(8) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44;
(9) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
(10) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44;
(11) a heavy chain variable region of the anti-CTLA4 immunoglobulin has the amino acid sequence set forth in SEQ ID NO: 2, and a light chain variable region of the anti-CTLA4 immunoglobulin has the amino acid sequence set forth in SEQ ID NO: 4; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(12) a heavy chain variable region of the anti-CTLA4 immunoglobulin has the amino acid sequence set forth in SEQ ID NO: 2, and a light chain variable region of the anti-CTLA4 immunoglobulin has the amino acid sequence set forth in SEQ ID NO: 4; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
(13) a heavy chain variable region of the anti-CTLA4 immunoglobulin has the amino acid sequence set forth in SEQ ID NO: 6, and a light chain variable region of the anti-CTLA4 immunoglobulin has the amino acid sequence set forth in SEQ ID NO: 8; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(14) a heavy chain variable region of the anti-CTLA4 immunoglobulin has the amino acid sequence set forth in SEQ ID NO: 6, and a light chain variable region of the anti-CTLA4 immunoglobulin has the amino acid sequence set forth in SEQ ID NO: 8; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
(15) a heavy chain variable region of the anti-CTLA4 immunoglobulin has the amino acid sequence set forth in SEQ ID NO: 10, and a light chain variable region of the anti-CTLA4 immunoglobulin has the amino acid sequence set forth in SEQ ID NO: 12; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(16) a heavy chain variable region of the anti-CTLA4 immunoglobulin has the amino acid sequence set forth in SEQ ID NO: 10, and a light chain variable region of the anti-CTLA4 immunoglobulin has the amino acid sequence set forth in SEQ ID NO: 12; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
(17) a heavy chain variable region of the anti-CTLA4 immunoglobulin has the amino acid sequence set forth in SEQ ID NO: 41, and a light chain variable region of the anti-CTLA4 immunoglobulin has the amino acid sequence set forth in SEQ ID NO: 42; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(18) a heavy chain variable region of the anti-CTLA4 immunoglobulin has the amino acid sequence set forth in SEQ ID NO: 43, and a light chain variable region of the anti-CTLA4 immunoglobulin has the amino acid sequence set forth in SEQ ID NO: 44; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(19) a heavy chain variable region of the anti-CTLA4 immunoglobulin has the amino acid sequence set forth in SEQ ID NO: 41, and a light chain variable region of the anti-CTLA4 immunoglobulin has the amino acid sequence set forth in SEQ ID NO: 42; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
and
(20) a heavy chain variable region of the anti-CTLA4 immunoglobulin has the amino acid sequence set forth in SEQ ID NO: 43, and a light chain variable region of the anti-CTLA4 immunoglobulin has the amino acid sequence set forth in SEQ ID NO: 44; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20.

7. The use according to any one of claims 1 to 6, wherein
a heavy chain of the anti-PD-1 antibody has the amino acid sequence set forth in SEQ ID NO: 40, and a light chain of the anti-PD-1 antibody has the amino acid sequence set forth in SEQ ID NO: 24; or
a heavy chain of the anti-PD-1 antibody has the amino acid sequence set forth in SEQ ID NO: 22, and a light chain of the anti-PD-1 antibody has the amino acid sequence set forth in SEQ ID NO: 24.

8. The use according to any one of claims 1 to 7, wherein the first protein functional region is linked to the second protein functional region either directly or via a linker; and/or the heavy chain variable region of the single chain antibody is linked to the light chain variable region of the single chain antibody either directly or via a linker;
preferably, the linker is independently the polypeptide set forth in SEQ ID NO: 47 or a polypeptide formed by concatenating a plurality of (e.g., 2, 3, 4, 5, or 6) polypeptides set forth in SEQ ID NO: 47;
preferably, the linker is independently a polypeptide having one or more glycines linked to the C-terminus of the polypeptide set forth in SEQ ID NO: 47 or a polypeptide formed by concatenating a plurality of (e.g., 2, 3, 4, 5, or 6) polypeptides set forth in SEQ ID NO: 47;
preferably, the first protein functional region and the second protein functional region are independently 1, 2, or more in number.

9. The use according to any one of claims 1 to 8, wherein
the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody;
a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26;
preferably, the first linker and the second linker both have the amino acid sequence set forth in SEQ ID NO: 26.

10. The use according to any one of claims 1 to 9, wherein
the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody;
a heavy chain of the anti-PD-1 immunoglobulin has the amino acid sequence set forth in SEQ ID NO: 22, and a light chain of the anti-PD-1 immunoglobulin has the amino acid sequence set forth in SEQ ID NO: 24;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26;
preferably, the first linker and the second linker both have the amino acid sequence set forth in SEQ ID NO: 26.

11. Use of a bispecific antibody or a pharmaceutical composition comprising the bispecific antibody in the preparation of a medicament for treating or preventing a malignant tumor of vulva, wherein the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody;
a heavy chain of the anti-PD-1 immunoglobulin has the amino acid sequence set forth in SEQ ID NO: 40, and a light chain of the anti-PD-1 immunoglobulin has the amino acid sequence set forth in SEQ ID NO: 24;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26;
preferably, the first linker and the second linker both have the amino acid sequence set forth in SEQ ID NO: 26.

12. The use according to any one of claims 1 to 11, wherein the malignant tumor of vulva is one or more selected from squamous cell carcinoma of vulva, malignant melanoma of vulva, basal cell carcinoma of vulva, verrucous carcinoma of vulva, Paget's disease of vulva, sarcoma of vulva, adenocarcinoma of vulva, and Bartholin gland carcinoma of vulva;
preferably, the malignant tumor of vulva is a recurrent malignant tumor or a metastatic malignant tumor of vulva;
preferably, the malignant tumor of vulva is an MSI-H/dMMR malignant tumor of vulva.

13. The use according to any one of claims 1 to 12, wherein, based on the mass of the bispecific antibody, the pharmaceutical composition has a unit dose of 100 mg-1000 mg, 200 mg-800 mg, 200 mg-500 mg, 300 mg-600 mg, 400 mg-500 mg, or 450 mg.

14. The use according to any one of claims 1 to 13, wherein the pharmaceutical composition further comprises one or more pharmaceutically acceptable auxiliary materials;
preferably, the pharmaceutical composition further comprises one or more antitumor chemotherapeutic drugs in effective amounts.

15. The use according to any one of claims 1 to 14, wherein the pharmaceutical composition is an injection; preferably, the pharmaceutical composition is administered by intravenous drip infusion, intravenous injection, subcutaneous injection, or intraperitoneal injection.

16. A bispecific antibody or a pharmaceutical composition comprising the bispecific antibody for use in treating or preventing a malignant tumor of vulva, wherein the bispecific antibody comprises:
a first protein functional region targeting PD-1 and
a second protein functional region targeting CTLA4,
wherein:
the first protein functional region is an anti-PD-1 antibody or an antigen-binding fragment thereof, and the anti-PD-1 antibody comprises a heavy chain variable region and a light chain variable region, wherein the heavy chain variable region comprises HCDR1 having the amino acid sequence set forth in SEQ ID NO: 27, HCDR2 having the amino acid sequence set forth in SEQ ID NO: 28, and HCDR3 having the amino acid sequence set forth in SEQ ID NO: 29; and the light chain variable region comprises LCDR1 having the amino acid sequence set forth in SEQ ID NO: 30, LCDR2 having the amino acid sequence set forth in SEQ ID NO: 31, and LCDR3 having the amino acid sequence set forth in SEQ ID NO: 32;
the second protein functional region is an anti-CTLA4 antibody or an antigen-binding fragment thereof, and the anti-CTLA4 antibody comprises a heavy chain variable region and a light chain variable region, wherein the heavy chain variable region comprises HCDR1 having the amino acid sequence set forth in SEQ ID NO: 33, HCDR2 having the amino acid sequence set forth in SEQ ID NO: 34, and HCDR3 having the amino acid sequence set forth in SEQ ID NO: 35; and the light chain variable region comprises LCDR1 having the amino acid sequence set forth in SEQ ID NO: 36, LCDR2 having the amino acid sequence set forth in SEQ ID NO: 37, and LCDR3 having the amino acid sequence set forth in SEQ ID NO: 38.

17. The bispecific antibody or the pharmaceutical composition comprising the bispecific antibody according to claim 16, wherein
the heavy chain variable region of the anti-PD-1 antibody has an amino acid sequence selected from SEQ ID NO: 14 and SEQ ID NO: 18, and the light chain variable region of the anti-PD-1 antibody has an amino acid sequence selected from SEQ ID NO: 16 and SEQ ID NO: 20;
preferably, the heavy chain variable region of the anti-PD-1 antibody has the amino acid sequence set forth in SEQ ID NO: 14, and the light chain variable region of the anti-PD-1 antibody has the amino acid sequence set forth in SEQ ID NO: 16; or
preferably, the heavy chain variable region of the anti-PD-1 antibody has the amino acid sequence set forth in SEQ ID NO: 18, and the light chain variable region of the anti-PD-1 antibody has the amino acid sequence set forth in SEQ ID NO: 20.

18. The bispecific antibody or the pharmaceutical composition comprising the bispecific antibody according to any one of claims 16 to 17, wherein
the heavy chain variable region of the anti-CTLA4 antibody has an amino acid sequence selected from SEQ ID NO: 2, SEQ ID NO: 6, SEQ ID NO: 10, SEQ ID NO: 41, and SEQ ID NO: 43, and the light chain variable region of the anti-CTLA4 antibody has an amino acid sequence selected from SEQ ID NO: 4, SEQ ID NO: 8, SEQ ID NO: 12, SEQ ID NO: 42, and SEQ ID NO: 44; preferably:
the heavy chain variable region of the anti-CTLA4 antibody has the amino acid sequence set forth in SEQ ID NO: 2, and the light chain variable region of the anti-CTLA4 antibody has the amino acid sequence set forth in SEQ ID NO: 4;
the heavy chain variable region of the anti-CTLA4 antibody has the amino acid sequence set forth in SEQ ID NO: 6, and the light chain variable region of the anti-CTLA4 antibody has the amino acid sequence set forth in SEQ ID NO: 8;
the heavy chain variable region of the anti-CTLA4 antibody has the amino acid sequence set forth in SEQ ID NO: 10, and the light chain variable region of the anti-CTLA4 antibody has the amino acid sequence set forth in SEQ ID NO: 12;
the heavy chain variable region of the anti-CTLA4 antibody has the amino acid sequence set forth in SEQ ID NO: 41, and the light chain variable region of the anti-CTLA4 antibody has the amino acid sequence set forth in SEQ ID NO: 42; or
the heavy chain variable region of the anti-CTLA4 antibody has the amino acid sequence set forth in SEQ ID NO: 43, and the light chain variable region of the anti-CTLA4 antibody has the amino acid sequence set forth in SEQ ID NO: 44.

19. The bispecific antibody or the pharmaceutical composition comprising the bispecific antibody according to any one of claims 16 to 18, wherein
the first protein functional region is an anti-PD-1 immunoglobulin, and the second protein functional region is an anti-CTLA4 single chain antibody;
preferably, the anti-CTLA4 single chain antibody is linked to the N-terminus or the C-terminus of a heavy chain of the anti-PD-1 immunoglobulin;
preferably, the anti-PD-1 immunoglobulin is of human IgG1 subtype;
preferably, according to the EU numbering system, a heavy chain constant region of the anti-PD-1 immunoglobulin has one of the following mutation combinations:
L234A and L235A;
L234A and G237A;
L235A and G237A; or
L234A, L235A, and G237A.

20. The bispecific antibody or the pharmaceutical composition comprising the bispecific antibody according to any one of claims 16 to 18, wherein
the first protein functional region is an anti-PD-1 single chain antibody, and the second protein functional region is an anti-CTLA4 immunoglobulin; preferably, the anti-PD-1 single chain antibody is linked to the N-terminus or
the C-terminus of a heavy chain of the anti-CTLA4 immunoglobulin;
preferably, the anti-CTLA4 immunoglobulin is of human IgG1 subtype;
preferably, according to the EU numbering system, a heavy chain constant region of the immunoglobulin has one of the following mutation combinations:
L234A and L235A;
L234A and G237A;
L235A and G237A; or
L234A, L235A, and G237A.

21. The bispecific antibody or the pharmaceutical composition comprising the bispecific antibody according to any one of claims 16 to 20, wherein
a heavy chain variable region of the anti-PD-1 immunoglobulin has an amino acid sequence selected from SEQ ID NO: 14 and SEQ ID NO: 18, and a light chain variable region of the anti-PD-1 immunoglobulin has an amino acid sequence selected from SEQ ID NO: 16 and SEQ ID NO: 20; and, a heavy chain variable region of the anti-CTLA4 single chain antibody has an amino acid sequence selected from SEQ ID NO: 2, SEQ ID NO: 6, SEQ ID NO: 10, SEQ ID NO: 41, and SEQ ID NO: 43, and a light chain variable region of the anti-CTLA4 single chain antibody has an amino acid sequence selected from SEQ ID NO: 4, SEQ ID NO: 8, SEQ ID NO: 12, SEQ ID NO: 42, and SEQ ID NO: 44;
or
a heavy chain variable region of the anti-CTLA4 immunoglobulin has an amino acid sequence selected from SEQ ID NO: 2, SEQ ID NO: 6, SEQ ID NO: 10, SEQ ID NO: 41, and SEQ ID NO: 43, and a light chain variable region of the anti-CTLA4 immunoglobulin has an amino acid sequence selected from SEQ ID NO: 4, SEQ ID NO: 8, SEQ ID NO: 12, SEQ ID NO: 42, and SEQ ID NO: 44; and, a heavy chain variable region of the anti-PD-1 single chain antibody has an amino acid sequence selected from SEQ ID NO: 14 and SEQ ID NO: 18, and a light chain variable region of the anti-PD-1 single chain antibody has an amino acid sequence selected from SEQ ID NO: 16 and SEQ ID NO: 20; preferably, the bispecific antibody is selected from any one of the following (1)-(20):
(1) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 2, and a light chain variable region of the anti-CTLA4 single chain antibody has the amino acid sequence set forth in SEQ ID NO: 4;
(2) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 6, and a light chain variable region of the anti-CTLA4 single chain antibody has the amino acid sequence set forth in SEQ ID NO: 8;
(3) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 10, and a light chain variable region of the anti-CTLA4 single chain antibody has the amino acid sequence set forth in SEQ ID NO: 12;
(4) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 2, and a light chain variable region of the anti-CTLA4 single chain antibody has the amino acid sequence set forth in SEQ ID NO: 4;
(5) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 6, and a light chain variable region of the anti-CTLA4 single chain antibody has the amino acid sequence set forth in SEQ ID NO: 8;
(6) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 10, and a light chain variable region of the anti-CTLA4 single chain antibody has the amino acid sequence set forth in SEQ ID NO: 12;
(7) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
(8) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44;
(9) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
(10) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44;
(11) a heavy chain variable region of the anti-CTLA4 immunoglobulin has the amino acid sequence set forth in SEQ ID NO: 2, and a light chain variable region of the anti-CTLA4 immunoglobulin has the amino acid sequence set forth in SEQ ID NO: 4; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(12) a heavy chain variable region of the anti-CTLA4 immunoglobulin has the amino acid sequence set forth in SEQ ID NO: 2, and a light chain variable region of the anti-CTLA4 immunoglobulin has the amino acid sequence set forth in SEQ ID NO: 4; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
(13) a heavy chain variable region of the anti-CTLA4 immunoglobulin has the amino acid sequence set forth in SEQ ID NO: 6, and a light chain variable region of the anti-CTLA4 immunoglobulin has the amino acid sequence set forth in SEQ ID NO: 8; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(14) a heavy chain variable region of the anti-CTLA4 immunoglobulin has the amino acid sequence set forth in SEQ ID NO: 6, and a light chain variable region of the anti-CTLA4 immunoglobulin has the amino acid sequence set forth in SEQ ID NO: 8; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
(15) a heavy chain variable region of the anti-CTLA4 immunoglobulin has the amino acid sequence set forth in SEQ ID NO: 10, and a light chain variable region of the anti-CTLA4 immunoglobulin has the amino acid sequence set forth in SEQ ID NO: 12; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(16) a heavy chain variable region of the anti-CTLA4 immunoglobulin has the amino acid sequence set forth in SEQ ID NO: 10, and a light chain variable region of the anti-CTLA4 immunoglobulin has the amino acid sequence set forth in SEQ ID NO: 12; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
(17) a heavy chain variable region of the anti-CTLA4 immunoglobulin has the amino acid sequence set forth in SEQ ID NO: 41, and a light chain variable region of the anti-CTLA4 immunoglobulin has the amino acid sequence set forth in SEQ ID NO: 42; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(18) a heavy chain variable region of the anti-CTLA4 immunoglobulin has the amino acid sequence set forth in SEQ ID NO: 43, and a light chain variable region of the anti-CTLA4 immunoglobulin has the amino acid sequence set forth in SEQ ID NO: 44; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(19) a heavy chain variable region of the anti-CTLA4 immunoglobulin has the amino acid sequence set forth in SEQ ID NO: 41, and a light chain variable region of the anti-CTLA4 immunoglobulin has the amino acid sequence set forth in SEQ ID NO: 42; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
and
(20) a heavy chain variable region of the anti-CTLA4 immunoglobulin has the amino acid sequence set forth in SEQ ID NO: 43, and a light chain variable region of the anti-CTLA4 immunoglobulin has the amino acid sequence set forth in SEQ ID NO: 44; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20.

22. The bispecific antibody or the pharmaceutical composition comprising the bispecific antibody according to any one of claims 16 to 21, wherein
a heavy chain of the anti-PD-1 antibody has the amino acid sequence set forth in SEQ ID NO: 40, and a light chain of the anti-PD-1 antibody has the amino acid sequence set forth in SEQ ID NO: 24; or
a heavy chain of the anti-PD-1 antibody has the amino acid sequence set forth in SEQ ID NO: 22, and a light chain of the anti-PD-1 antibody has the amino acid sequence set forth in SEQ ID NO: 24.

23. The bispecific antibody or the pharmaceutical composition comprising the bispecific antibody according to any one of claims 16 to 22, wherein the first protein functional region is linked to the second protein functional region either directly or via a linker; and/or the heavy chain variable region of the single chain antibody is linked to the light chain variable region of the single chain antibody either directly or via a linker;
preferably, the linker is independently the polypeptide set forth in SEQ ID NO: 47 or a polypeptide formed by concatenating a plurality of (e.g., 2, 3, 4, 5, or 6) polypeptides set forth in SEQ ID NO: 47;
preferably, the linker is independently a polypeptide having one or more glycines linked to the C-terminus of the polypeptide set forth in SEQ ID NO: 47 or a polypeptide formed by concatenating a plurality of (e.g., 2, 3, 4, 5, or 6) polypeptides set forth in SEQ ID NO: 47;
preferably, the first protein functional region and the second protein functional region are independently 1, 2, or more in number.

24. The bispecific antibody or the pharmaceutical composition comprising the bispecific antibody according to any one of claims 16 to 23, wherein
the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody;
a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26;
preferably, the first linker and the second linker both have the amino acid sequence set forth in SEQ ID NO: 26.

25. The bispecific antibody or the pharmaceutical composition comprising the bispecific antibody according to any one of claims 16 to 24, wherein
the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody;
a heavy chain of the anti-PD-1 immunoglobulin has the amino acid sequence set forth in SEQ ID NO: 22, and a light chain of the anti-PD-1 immunoglobulin has the amino acid sequence set forth in SEQ ID NO: 24;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26;
preferably, the first linker and the second linker both have the amino acid sequence set forth in SEQ ID NO: 26.

26. A bispecific antibody or a pharmaceutical composition comprising the bispecific antibody for use in treating or preventing a malignant tumor of vulva, wherein the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody;
a heavy chain of the anti-PD-1 immunoglobulin has the amino acid sequence set forth in SEQ ID NO: 40, and a light chain of the anti-PD-1 immunoglobulin has the amino acid sequence set forth in SEQ ID NO: 24;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26;
preferably, the first linker and the second linker both have the amino acid sequence set forth in SEQ ID NO: 26.

27. The bispecific antibody or the pharmaceutical composition comprising the bispecific antibody according to any one of claims 16 to 26, wherein the malignant tumor of vulva is one or more selected from squamous cell carcinoma of vulva, malignant melanoma of vulva, basal cell carcinoma of vulva, verrucous carcinoma of vulva, Paget's disease of vulva, sarcoma of vulva, adenocarcinoma of vulva, and Bartholin gland carcinoma of vulva;
preferably, the malignant tumor of vulva is a recurrent malignant tumor or a metastatic malignant tumor of vulva;
preferably, the malignant tumor of vulva is an MSI-H/dMMR malignant tumor of vulva.

28. The bispecific antibody or the pharmaceutical composition comprising the bispecific antibody according to any one of claims 16 to 27, wherein, based on the mass of the bispecific antibody, the pharmaceutical composition has a unit dose of 100 mg-1000 mg, 200 mg-800 mg, 200 mg-500 mg, 300 mg-600 mg, 400 mg-500 mg, or 450 mg.

29. The bispecific antibody or the pharmaceutical composition comprising the bispecific antibody according to any one of claims 16 to 28, wherein the pharmaceutical composition further comprises one or more pharmaceutically acceptable auxiliary materials;
preferably, the pharmaceutical composition further comprises one or more antitumor chemotherapeutic drugs in effective amounts.

30. The bispecific antibody or the pharmaceutical composition comprising the bispecific antibody according to any one of claims 16 to 29, wherein the pharmaceutical composition is an injection; preferably, the pharmaceutical composition is administered by intravenous drip infusion, intravenous injection, subcutaneous injection, or intraperitoneal injection.

31. A method for treating or preventing a malignant tumor of vulva, comprising a step of administering to a subject in need an effective amount of a bispecific antibody or a pharmaceutical composition comprising the bispecific antibody, wherein the bispecific antibody comprises:
a first protein functional region targeting PD-1 and
a second protein functional region targeting CTLA4,
wherein:
the first protein functional region is an anti-PD-1 antibody or an antigen-binding fragment thereof, and the anti-PD-1 antibody comprises a heavy chain variable region and a light chain variable region, wherein the heavy chain variable region comprises HCDR1 having the amino acid sequence set forth in SEQ ID NO: 27, HCDR2 having the amino acid sequence set forth in SEQ ID NO: 28, and HCDR3 having the amino acid sequence set forth in SEQ ID NO: 29; and the light chain variable region comprises LCDR1 having the amino acid sequence set forth in SEQ ID NO: 30, LCDR2 having the amino acid sequence set forth in SEQ ID NO: 31, and LCDR3 having the amino acid sequence set forth in SEQ ID NO: 32;
the second protein functional region is an anti-CTLA4 antibody or an antigen-binding fragment thereof, and the anti-CTLA4 antibody comprises a heavy chain variable region and a light chain variable region, wherein the heavy chain variable region comprises HCDR1 having the amino acid sequence set forth in SEQ ID NO: 33, HCDR2 having the amino acid sequence set forth in SEQ ID NO: 34, and HCDR3 having the amino acid sequence set forth in SEQ ID NO: 35; and the light chain variable region comprises LCDR1 having the amino acid sequence set forth in SEQ ID NO: 36, LCDR2 having the amino acid sequence set forth in SEQ ID NO: 37, and LCDR3 having the amino acid sequence set forth in SEQ ID NO: 38.

32. The method for treating or preventing a malignant tumor of vulva according to claim 31, wherein
the heavy chain variable region of the anti-PD-1 antibody has an amino acid sequence selected from SEQ ID NO: 14 and SEQ ID NO: 18, and the light chain variable region of the anti-PD-1 antibody has an amino acid sequence selected from SEQ ID NO: 16 and SEQ ID NO: 20;
preferably, the heavy chain variable region of the anti-PD-1 antibody has the amino acid sequence set forth in SEQ ID NO: 14, and the light chain variable region of the anti-PD-1 antibody has the amino acid sequence set forth in SEQ ID NO: 16; or
preferably, the heavy chain variable region of the anti-PD-1 antibody has the amino acid sequence set forth in SEQ ID NO: 18, and the light chain variable region of the anti-PD-1 antibody has the amino acid sequence set forth in SEQ ID NO: 20.

33. The method for treating or preventing a malignant tumor of vulva according to any one of claims 31 to 32, wherein
the heavy chain variable region of the anti-CTLA4 antibody has an amino acid sequence selected from SEQ ID NO: 2, SEQ ID NO: 6, SEQ ID NO: 10, SEQ ID NO: 41, and SEQ ID NO: 43, and the light chain variable region of the anti-CTLA4 antibody has an amino acid sequence selected from SEQ ID NO: 4, SEQ ID NO: 8, SEQ ID NO: 12, SEQ ID NO: 42, and SEQ ID NO: 44;
preferably:
the heavy chain variable region of the anti-CTLA4 antibody has the amino acid sequence set forth in SEQ ID NO: 2, and the light chain variable region of the anti-CTLA4 antibody has the amino acid sequence set forth in SEQ ID NO: 4;
the heavy chain variable region of the anti-CTLA4 antibody has the amino acid sequence set forth in SEQ ID NO: 6, and the light chain variable region of the anti-CTLA4 antibody has the amino acid sequence set forth in SEQ ID NO: 8;
the heavy chain variable region of the anti-CTLA4 antibody has the amino acid sequence set forth in SEQ ID NO: 10, and the light chain variable region of the anti-CTLA4 antibody has the amino acid sequence set forth in SEQ ID NO: 12;
the heavy chain variable region of the anti-CTLA4 antibody has the amino acid sequence set forth in SEQ ID NO: 41, and the light chain variable region of the anti-CTLA4 antibody has the amino acid sequence set forth in SEQ ID NO: 42; or
the heavy chain variable region of the anti-CTLA4 antibody has the amino acid sequence set forth in SEQ ID NO: 43, and the light chain variable region of the anti-CTLA4 antibody has the amino acid sequence set forth in SEQ ID NO: 44.

34. The method for treating or preventing a malignant tumor of vulva according to any one of claims 31 to 33, wherein
the first protein functional region is an anti-PD-1 immunoglobulin, and the second protein functional region is an anti-CTLA4 single chain antibody;
preferably, the anti-CTLA4 single chain antibody is linked to the N-terminus or the C-terminus of a heavy chain of the anti-PD-1 immunoglobulin;
preferably, the anti-PD-1 immunoglobulin is of human IgG1 subtype;
preferably, according to the EU numbering system, a heavy chain constant region of the anti-PD-1 immunoglobulin has one of the following mutation combinations:
L234A and L235A;
L234A and G237A;
L235A and G237A; or
L234A, L235A, and G237A.

35. The method for treating or preventing a malignant tumor of vulva according to any one of claims 31 to 33, wherein
the first protein functional region is an anti-PD-1 single chain antibody, and the second protein functional region is an anti-CTLA4 immunoglobulin; preferably, the anti-PD-1 single chain antibody is linked to the N-terminus or
the C-terminus of a heavy chain of the anti-CTLA4 immunoglobulin;
preferably, the anti-CTLA4 immunoglobulin is of human IgG1 subtype; preferably, according to the EU numbering system, a heavy chain constant region of the immunoglobulin has one of the following mutation combinations:
L234A and L235A;
L234A and G237A;
L235A and G237A; or
L234A, L235A, and G237A.

36. The method for treating or preventing a malignant tumor of vulva according to any one of claims 31 to 35, wherein
a heavy chain variable region of the anti-PD-1 immunoglobulin has an amino acid sequence selected from SEQ ID NO: 14 and SEQ ID NO: 18, and a light chain variable region of the anti-PD-1 immunoglobulin has an amino acid sequence selected from SEQ ID NO: 16 and SEQ ID NO: 20; and, a heavy chain variable region of the anti-CTLA4 single chain antibody has an amino acid sequence selected from SEQ ID NO: 2, SEQ ID NO: 6, SEQ ID NO: 10, SEQ ID NO: 41, and SEQ ID NO: 43, and a light chain variable region of the anti-CTLA4 single chain antibody has an amino acid sequence selected from SEQ ID NO: 4, SEQ ID NO: 8, SEQ ID NO: 12, SEQ ID NO: 42, and SEQ ID NO: 44;
or
a heavy chain variable region of the anti-CTLA4 immunoglobulin has an amino acid sequence selected from SEQ ID NO: 2, SEQ ID NO: 6, SEQ ID NO: 10, SEQ ID NO: 41, and SEQ ID NO: 43, and a light chain variable region of the anti-CTLA4 immunoglobulin has an amino acid sequence selected from SEQ ID NO: 4, SEQ ID NO: 8, SEQ ID NO: 12, SEQ ID NO: 42, and SEQ ID NO: 44; and, a heavy chain variable region of the anti-PD-1 single chain antibody has an amino acid sequence selected from SEQ ID NO: 14 and SEQ ID NO: 18, and a light chain variable region of the anti-PD-1 single chain antibody has an amino acid sequence selected from SEQ ID NO: 16 and SEQ ID NO: 20; preferably, the bispecific antibody is selected from any one of the following (1)-(20):
(1) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 2, and a light chain variable region of the anti-CTLA4 single chain antibody has the amino acid sequence set forth in SEQ ID NO: 4;
(2) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 6, and a light chain variable region of the anti-CTLA4 single chain antibody has the amino acid sequence set forth in SEQ ID NO: 8;
(3) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 10, and a light chain variable region of the anti-CTLA4 single chain antibody has the amino acid sequence set forth in SEQ ID NO: 12;
(4) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 2, and a light chain variable region of the anti-CTLA4 single chain antibody has the amino acid sequence set forth in SEQ ID NO: 4;
(5) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 6, and a light chain variable region of the anti-CTLA4 single chain antibody has the amino acid sequence set forth in SEQ ID NO: 8;
(6) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 10, and a light chain variable region of the anti-CTLA4 single chain antibody has the amino acid sequence set forth in SEQ ID NO: 12;
(7) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
(8) a heavy chain variable region of the anti-PD-1 immunoglobulin has the amino acid sequence set forth in SEQ ID NO: 14, and a light chain variable region of the anti-PD-1 immunoglobulin has the amino acid sequence set forth in SEQ ID NO: 16; and, a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44;
(9) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
(10) a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20; and, a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44;
(11) a heavy chain variable region of the anti-CTLA4 immunoglobulin has the amino acid sequence set forth in SEQ ID NO: 2, and a light chain variable region of the anti-CTLA4 immunoglobulin has the amino acid sequence set forth in SEQ ID NO: 4; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(12) a heavy chain variable region of the anti-CTLA4 immunoglobulin has the amino acid sequence set forth in SEQ ID NO: 2, and a light chain variable region of the anti-CTLA4 immunoglobulin has the amino acid sequence set forth in SEQ ID NO: 4; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
(13) a heavy chain variable region of the anti-CTLA4 immunoglobulin has the amino acid sequence set forth in SEQ ID NO: 6, and a light chain variable region of the anti-CTLA4 immunoglobulin has the amino acid sequence set forth in SEQ ID NO: 8; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(14) a heavy chain variable region of the anti-CTLA4 immunoglobulin has the amino acid sequence set forth in SEQ ID NO: 6, and a light chain variable region of the anti-CTLA4 immunoglobulin has the amino acid sequence set forth in SEQ ID NO: 8; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
(15) a heavy chain variable region of the anti-CTLA4 immunoglobulin has the amino acid sequence set forth in SEQ ID NO: 10, and a light chain variable region of the anti-CTLA4 immunoglobulin has the amino acid sequence set forth in SEQ ID NO: 12; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(16) a heavy chain variable region of the anti-CTLA4 immunoglobulin has the amino acid sequence set forth in SEQ ID NO: 10, and a light chain variable region of the anti-CTLA4 immunoglobulin has the amino acid sequence set forth in SEQ ID NO: 12; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
(17) a heavy chain variable region of the anti-CTLA4 immunoglobulin has the amino acid sequence set forth in SEQ ID NO: 41, and a light chain variable region of the anti-CTLA4 immunoglobulin has the amino acid sequence set forth in SEQ ID NO: 42; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(18) a heavy chain variable region of the anti-CTLA4 immunoglobulin has the amino acid sequence set forth in SEQ ID NO: 43, and a light chain variable region of the anti-CTLA4 immunoglobulin has the amino acid sequence set forth in SEQ ID NO: 44; and, a heavy chain variable region of the anti-PD-1 single chain antibody has the amino acid sequence set forth in SEQ ID NO: 14, and a light chain variable region of the anti-PD-1 single chain antibody has the amino acid sequence set forth in SEQ ID NO: 16;
(19) a heavy chain variable region of the anti-CTLA4 immunoglobulin has the amino acid sequence set forth in SEQ ID NO: 41, and a light chain variable region of the anti-CTLA4 immunoglobulin has the amino acid sequence set forth in SEQ ID NO: 42; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20;
and
(20) a heavy chain variable region of the anti-CTLA4 immunoglobulin has the amino acid sequence set forth in SEQ ID NO: 43, and a light chain variable region of the anti-CTLA4 immunoglobulin has the amino acid sequence set forth in SEQ ID NO: 44; and, a heavy chain variable region of the anti-PD-1 single chain antibody has the amino acid sequence set forth in SEQ ID NO: 18, and a light chain variable region of the anti-PD-1 single chain antibody has the amino acid sequence set forth in SEQ ID NO: 20.

37. The method for treating or preventing a malignant tumor of vulva according to any one of claims 31 to 36, wherein
a heavy chain of the anti-PD-1 antibody has the amino acid sequence set forth in SEQ ID NO: 40, and a light chain of the anti-PD-1 antibody has the amino acid sequence set forth in SEQ ID NO: 24; or
a heavy chain of the anti-PD-1 antibody has the amino acid sequence set forth in SEQ ID NO: 22, and a light chain of the anti-PD-1 antibody has the amino acid sequence set forth in SEQ ID NO: 24.

38. The method for treating or preventing a malignant tumor of vulva according to any one of claims 31 to 37, wherein the first protein functional region is linked to the second protein functional region either directly or via a linker; and/or the heavy chain variable region of the single chain antibody is linked to the light chain variable region of the single chain antibody either directly or via a linker;
preferably, the linker is independently the polypeptide set forth in SEQ ID NO: 47 or a polypeptide formed by concatenating a plurality of (e.g., 2, 3, 4, 5, or 6) polypeptides set forth in SEQ ID NO: 47;
preferably, the linker is independently a polypeptide having one or more glycines linked to the C-terminus of the polypeptide set forth in SEQ ID NO: 47 or a polypeptide formed by concatenating a plurality of (e.g., 2, 3, 4, 5, or 6) polypeptides set forth in SEQ ID NO: 47;
preferably, the first protein functional region and the second protein functional region are independently 1, 2, or more in number.

39. The method for treating or preventing a malignant tumor of vulva according to any one of claims 31 to 38, wherein
the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody;
a heavy chain variable region of the anti-PD-1 immunoglobulin has the amino acid sequence set forth in SEQ ID NO: 18, and a light chain variable region of the anti-PD-1 immunoglobulin has the amino acid sequence set forth in SEQ ID NO: 20;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26;
preferably, the first linker and the second linker both have the amino acid sequence set forth in SEQ ID NO: 26.

40. The method for treating or preventing a malignant tumor of vulva according to any one of claims 31 to 39, wherein
the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody;
a heavy chain of the anti-PD-1 immunoglobulin has the amino acid sequence set forth in SEQ ID NO: 22, and a light chain of the anti-PD-1 immunoglobulin has the amino acid sequence set forth in SEQ ID NO: 24;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26;
preferably, the first linker and the second linker both have the amino acid sequence set forth in SEQ ID NO: 26.

41. A method for treating or preventing a malignant tumor of vulva, comprising a step of administering to a subject in need an effective amount of a bispecific antibody or a pharmaceutical composition comprising the bispecific antibody, wherein the bispecific antibody comprises:
a first protein functional region targeting PD-1 and
a second protein functional region targeting CTLA4,
the first protein functional region is 1 in number, and the second protein functional region is 2 in number;
wherein the first protein functional region is an anti-PD-1 immunoglobulin, and the second protein functional region is an anti-CTLA4 single chain antibody;
a heavy chain of the anti-PD-1 immunoglobulin has the amino acid sequence set forth in SEQ ID NO: 40, and a light chain of the anti-PD-1 immunoglobulin has the amino acid sequence set forth in SEQ ID NO: 24;
a heavy chain variable region of the anti-CTLA4 single chain antibody has the amino acid sequence set forth in SEQ ID NO: 43, and a light chain variable region of the anti-CTLA4 single chain antibody has the amino acid sequence set forth in SEQ ID NO: 44; or, a heavy chain variable region of the anti-CTLA4 single chain antibody has the amino acid sequence set forth in SEQ ID NO: 41, and a light chain variable region of the anti-CTLA4 single chain antibody has the amino acid sequence set forth in SEQ ID NO: 42;
the anti-CTLA4 single chain antibody is linked to the C-terminus of a heavy chain of the anti-PD-1 immunoglobulin;
the first protein functional region is linked to the second protein functional region via a first linker, and the heavy chain variable region of the anti-CTLA4 single chain antibody is linked to the light chain variable region of the anti-CTLA4 single chain antibody via a second linker; the first linker and the second linker are identical or different;
preferably, the first linker and the second linker each have an amino acid sequence independently selected from SEQ ID NO: 25 and SEQ ID NO: 26;
preferably, the first linker and the second linker both have the amino acid sequence set forth in SEQ ID NO: 26.

42. The method for treating or preventing a malignant tumor of vulva according to any one of claims 31 to 41, wherein the malignant tumor of vulva is one or more selected from squamous cell carcinoma of vulva, malignant melanoma of vulva, basal cell carcinoma of vulva, verrucous carcinoma of vulva, Paget's disease of vulva, sarcoma of vulva, adenocarcinoma of vulva, and Bartholin gland carcinoma of vulva;
preferably, the malignant tumor of vulva is a recurrent malignant tumor or a metastatic malignant tumor of vulva;
preferably, the malignant tumor of vulva is an MSI-H/dMMR malignant tumor of vulva.

43. The method for treating or preventing a malignant tumor of vulva according to any one of claims 31 to 42, wherein, based on the mass of the bispecific antibody, the pharmaceutical composition has a unit dose of 100 mg-1000 mg, 200 mg-800 mg, 200 mg-500 mg, 300 mg-600 mg, 400 mg-500 mg, or 450 mg.

44. The method for treating or preventing a malignant tumor of vulva according to any one of claims 31 to 43, wherein the pharmaceutical composition further comprises one or more pharmaceutically acceptable auxiliary materials; preferably, the pharmaceutical composition further comprises one or more antitumor chemotherapeutic drugs in effective amounts.

45. The method for treating or preventing a malignant tumor of vulva according to any one of claims 31 to 44, wherein the pharmaceutical composition is an injection; preferably, the pharmaceutical composition is administered by intravenous drip infusion, intravenous injection, subcutaneous injection, or intraperitoneal injection.
